# EUROPEAN PATENT APPLICATION

(11) **EP 2 044 943 A1**
(43) Date of publication of application: **08.04.2009**
(21) Application number: 07117920.4
(22) Date of filing: 04.10.2007
(51) Int. Cl.: A61K 31/566, A61K 31/568, A61P 15/12

(54) **Pharmaceutical compositions comprising esterified estrogens and methyltestosterone and method of using the same**

(71) Applicant: Solvay Pharmaceuticals, Inc., Marietta, GA 30062 (US)
(72) Inventor: Clark, Bradley A., Marietta, GA 30062 (US); Dubash, Darius D., Marietta, GA 30062 (US); Curry, Jr., Paul D., Marietta, GA 30062 (US)
(74) Representative: Gosmann, Martin

(57) **Abstract**

The present invention relates generally to pharmaceutical compositions comprising esterified estrogens, methyltestosterone and one or more pharmaceutical excipients. Methods for preparing and using such compositions are also provided.

## Description

### FIELD OF THE INVENTION

Described herein are pharmaceutical compositions comprising esterified estrogens and methyltestosterone and methods of preparing and using such compositions.

### BACKGROUND

Estrogens represent an important clinical modality in the treatment of vasomotor symptoms associated with menopause in women. Despite this clinical utility, some questions have been raised relating to the side effect profile of clinical estrogens. For example, the Women's Health Initiative (WHI) study reported increased risks of myocardial infarction, stroke, invasive breast cancer, pulmonary emboli, and deep vein thrombosis in postmenopausal women during 5 years of treatment with oral conjugated estrogens combined with medroxyprogesterone acetate relative to placebo.

Because of the above risks, it is desirable that any estrogen formulation be administered to a subject at the lowest effective dose and for the shortest duration consistent with treatment goals and risks for individual women.

If compositions, methods of treatment, methods of manufacture or other advances could be developed that provide improved estrogen therapy, a significant advance in the art would result.

### SUMMARY

In one embodiment, the present disclosure provides oral pharmaceutical compositions comprising esterified estrogens, methyltestosterone and one or more pharmaceutical excipients. The active agents may be co-administered as separate dosage forms or in one dosage form.

In another embodiment, the disclosure provides compositions comprising esterified estrogens, methyltestosterone and a moisture scavenger (or "moisture scavenging agent"). In yet another embodiment, the disclosure provides compositions comprising esterified estrogens, methyltestosterone, a diluent, an alkalizing agent, and a moisture scavenger.

In still another embodiment, the present disclosure provides a pharmaceutical composition comprising esterified estrogens, methyltestosterone and a moisture scavenging agent, wherein the esterified estrogens comprise an initial amount of sodium equilin sulfate and an initial amount of sodium estrone sulfate and wherein upon storage of the composition in an open container maintained at about 35 °C to about 45 °C and about 70% to about 80% relative humidity for a period of about 25 to about 30 days, about 85% to about 95% of the initial amount of sodium equilin sulfate is still present in the composition. For example, the embodiments disclosed herein may be stored in an open container maintained at about 40 °C and about 75% relative humidity for a period of about 28 days and about 91.4% of the initial amount of sodium equilin sulfate is still present in the composition.

Also disclosed herein are methods for preparing compositions of the embodiments described herein.

In another embodiment, the disclosure provides methods for treating and/or preventing hormone-mediated diseases and/or disorders in a subject in need thereof, comprising administering a therapeutically effective amount of a composition comprising esterified estrogens and methyltestosterone to the subject. In one embodiment, the hormone-related disorder is an estrogen-related disorder, and synergistic amounts of esterified estrogens and methyltestosterone are co-administered.

Other objects, features and advantages will be set forth in the Detailed Description that follows, and in part will be apparent from the description or may be learned by practice of the embodiments disclosed herein. These objects and advantages will be realized and attained by the processes and compositions particularly pointed out in the written description and claims hereof.

### BRIEF DESCRIPTION OF THE DRAWINGS

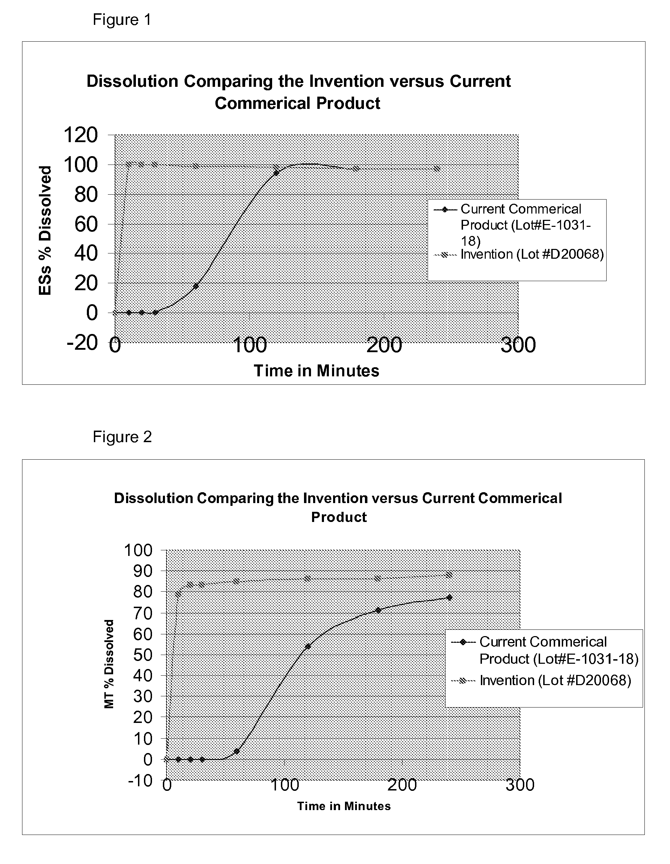

**Figure 1** shows a comparison of the esterified estrogens dissolution performance of the invention and of the current Estratest® (esterified estrogens / methyltestosterone) tablets. Dissolution conditions were: USP Dissolution Method 1 (baskets), 50 RPM, 10% Simulated Intestinal Fluid.

**Figure 2** shows a comparison of the methyltestosterone dissolution performance of the invention and of the current Estratest® (esterified estrogens / methyltestosterone) tablets. Dissolution conditions were: USP Dissolution Method 1 (baskets), 50 RPM, 10% Simulated Intestinal Fluid.

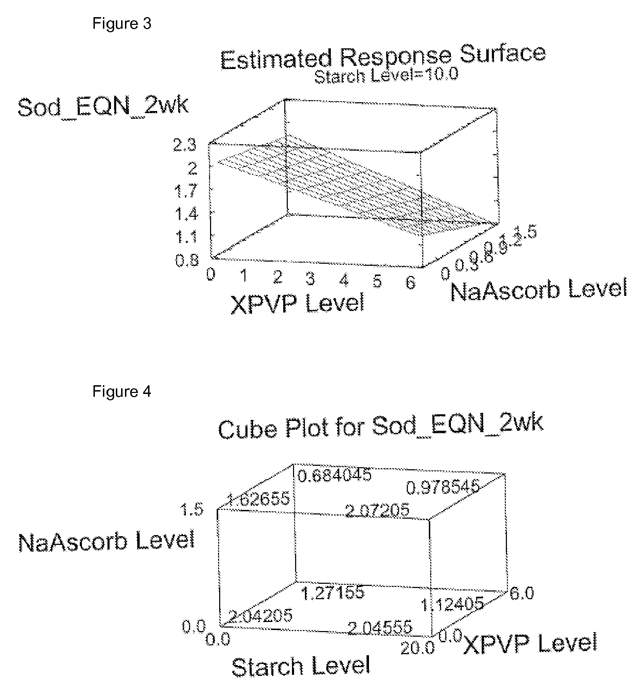

**Figure 3** shows a response plot for sodium equilenin sulfate formation in 2-week open-dish samples as a function of crospovidone level and sodium ascorbate content, holding starch content at the median level. Samples were stored at 40°C/75%RH conditions. Factor values are expressed in mg/tablet; sodium equilenin sulfate is in percent (%).

**Figure 4** shows a cube plot for sodium equilenin sulfate formation in 2-week open-dish samples as a function of starch, crospovidone and sodium ascorbate levels. Samples were stored at 40°C/75%RH conditions. Factor values are expressed in mg/tablet; sodium equilenin sulfate is in percent (%).

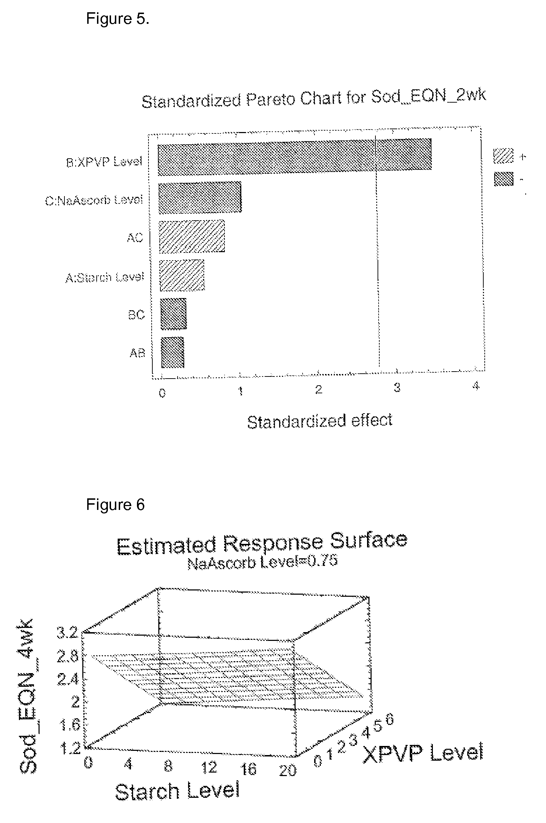

**Figure 5** shows a Pareto plot of effect level on sodium equilenin sulfate formation in 2-week open-dish samples as a function of starch, crospovidone and sodium ascorbate levels. Samples were stored at 40°C/75%RH conditions. Factor values are expressed in mg/tablet; sodium equilenin sulfate is in percent (%).

**Figure 6** shows a response plot for sodium equilenin sulfate formation in 4-week open-dish samples as a function of starch content and crospovidone level, holding sodium ascorbate level at the median level. Samples were stored at 40°C/75%RH conditions. Factor values are expressed in mg/tablet; sodium equilenin sulfate is in percent (%).

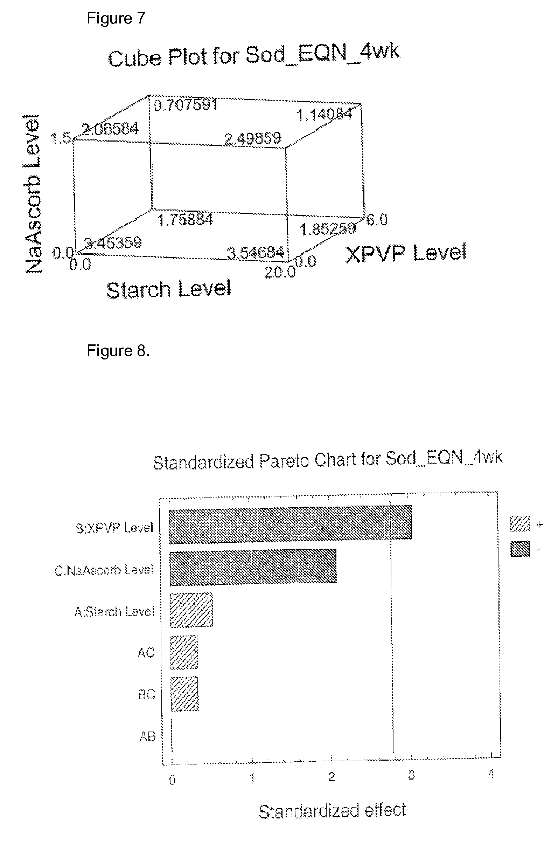

**Figure 7** shows a cube plot for sodium equilenin sulfate formation in 4-week open-dish samples as a function of starch, crospovidone and sodium ascorbate levels. Samples were stored at 40°C/75%RH conditions. Factor values are expressed in mg/tablet; sodium equilenin sulfate is in percent (%).

**Figure 8** shows a Pareto plot of effect level on sodium equilenin sulfate formation in 4-week open-dish samples as a function of starch, crospovidone and sodium ascorbate levels. Samples were stored at 40°C/75%RH conditions. Factor values are expressed in mg/tablet; sodium equilenin sulfate is in percent (%).

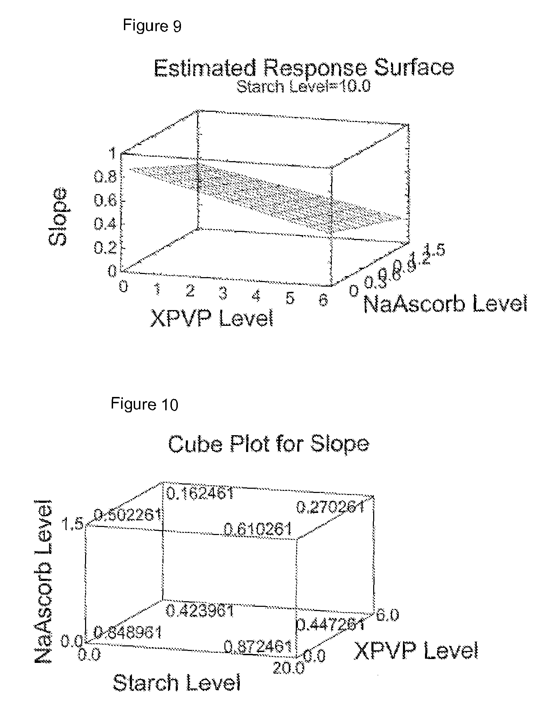

**Figure 9** shows a response plot of slope for rate of sodium equilenin sulfate formation in 4-week open-dish samples as a function of crospovidone and sodium ascorbate levels, holding starch level at the median value. Samples were stored at 40°C/75%RH conditions. Values for factors are expressed in mg/tablet; slope expressed as percent/week.

**Figure 10** shows a cube plot of slope for rate of sodium equilenin sulfate formation in 4-week open-dish samples as a function of starch, crospovidone and sodium ascorbate levels. Samples were stored at 40°C/75%RH conditions. Values for factors are expressed in mg/tablet slope expressed as percent/week.

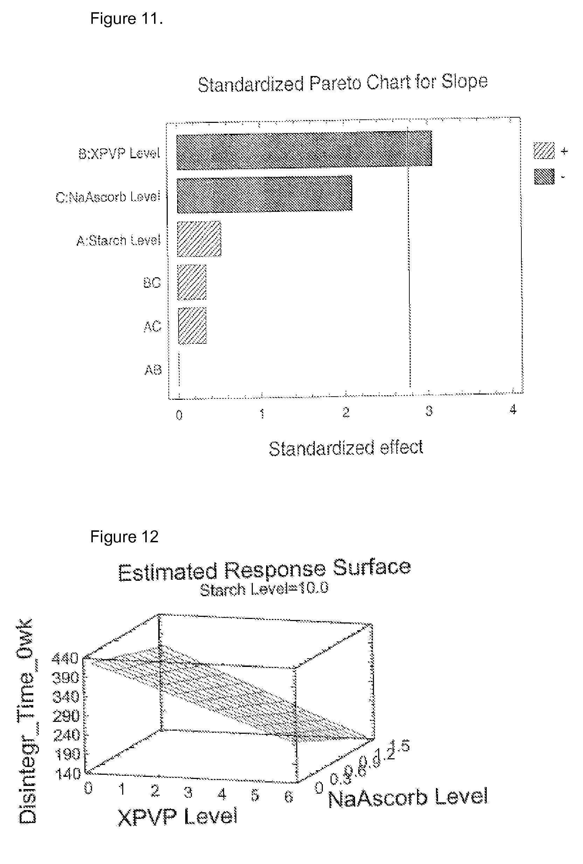

**Figure 11** shows a Pareto plot of effect level on the rate of formation (slope) of sodium equilenin sulfate in 4-week open-dish samples as a function of starch, crospovidone and sodium ascorbate levels. Samples were stored at 40°C/75%RH conditions. Values for factors are expressed in mg/tablet slope expressed as percent/week.

**Figure 12** shows a response plot for disintegration time of esterified estrogens/methyltestosterone uncoated tablets for 0-week (To) open-dish samples as a function of crospovidone content and sodium ascorbate level, holding starch content at the median level. Samples were stored at 40°C/75%RH conditions. Values for factors are expressed in mg/tablet; disintegration times are given in seconds.

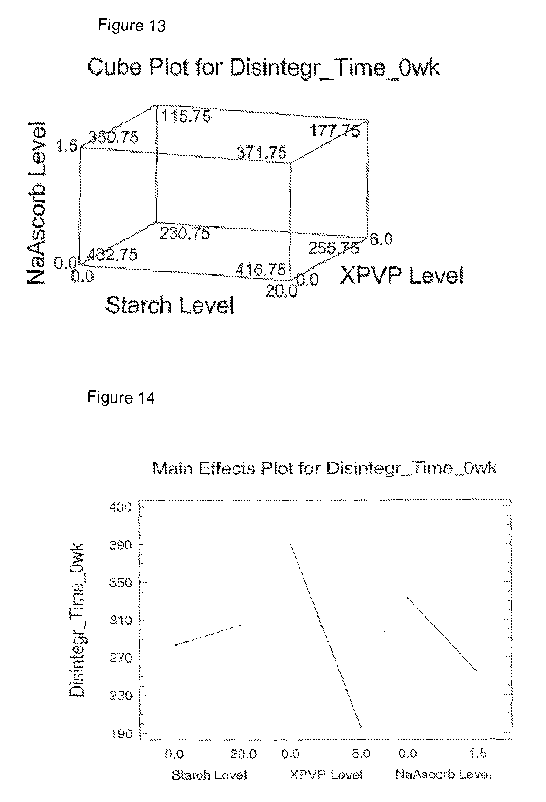

**Figure 13** shows a cube plot for disintegration time of esterified estrogens/methyltestosterone uncoated tablets for 0-week (To) open-dish samples as a function of starch, crospovidone and sodium ascorbate levels. Samples were stored at 40°C/75%RH conditions. Values for factors are expressed in mg/tablet; esterified estrogens/methyltestosterone disintegration values are given in seconds.

**Figure 14** shows a main effects plot for disintegration time of esterified estrogens/methyltestosterone uncoated tablets for 0-week (To) open-dish samples as a function of starch, crospovidone and sodium ascorbate levels. Samples were stored at 40°C/75%RH conditions. Values for factors are expressed in mg/tablet; esterified estrogens/methyltestosterone disintegration values are given in seconds.

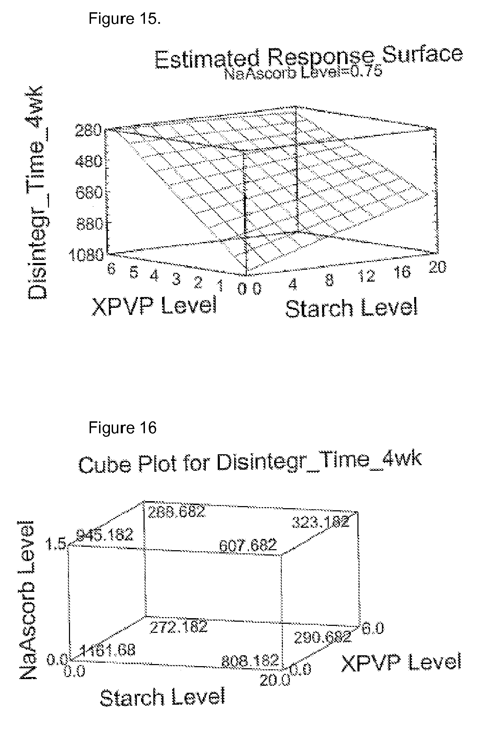

**Figure 15** shows a response plot for disintegration time of esterified estrogens/methyltestosterone uncoated tablets for 4-week open-dish samples as a function of crospovidone content and starch level, holding sodium ascorbate content at the median level. Samples were stored at 40°C/75%RH conditions. Values for factors are expressed in mg/tablet; disintegration times are given in seconds.

**Figure 16** shows a cube plot for disintegration time of esterified estrogens/methyltestosterone uncoated tablets for 4-week open-dish samples as a function of starch, crospovidone and sodium ascorbate levels. Samples were stored at 40°C/75%RH conditions. Values for factors are expressed in mg/tablet; esterified estrogens/methyltestosterone disintegration values are given in seconds.

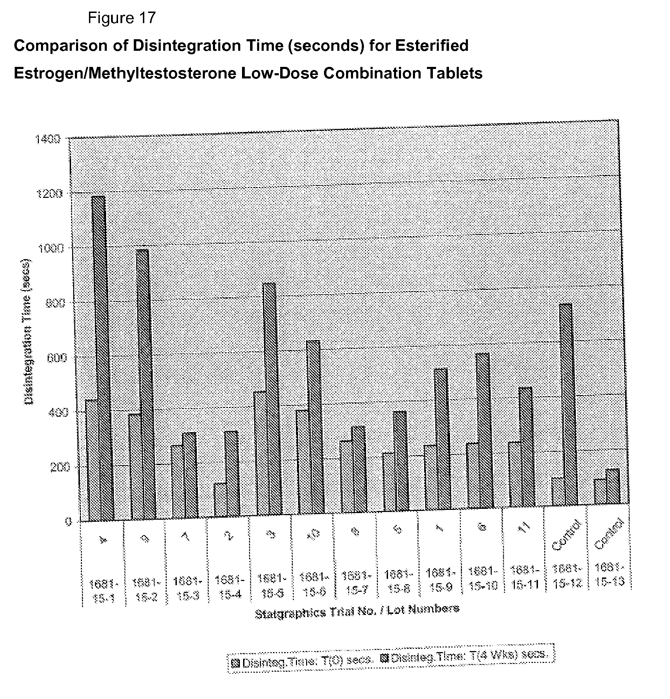

**Figure 17** shows a Comparison of Disintegration Times (seconds) for EE/MT Low-Dose Combination Tablets of the present disclosure. Formulation numbers correspond with those of Table 4.

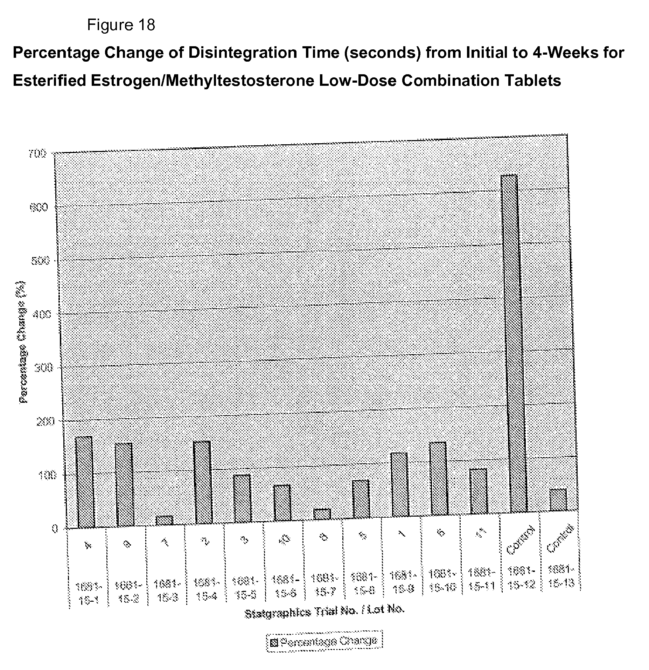

**Figure 18** shows Percentage Change of Disintegration Time (seconds) from Initial to 4-Weeks for EE/MT Low-Dose Combination Tablets of the present disclosure. The formulation numbers correspond with those of Table 4.

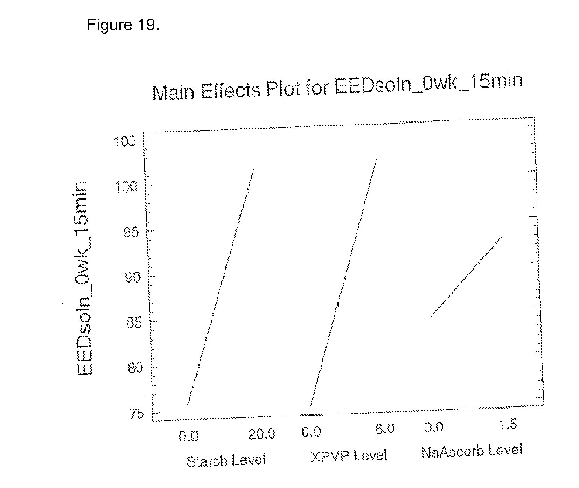

**Figure 19** shows a main effects plot for percent release of esterified estrogens in dissolution tests for time 0 open-dish samples as a function of starch content and crospovidone level, holding sodium ascorbate content at the median level. Samples were stored at 40°C/75%RH conditions. Values for factors are expressed in mg/tablet; esterified estrogens dissolution values are percent released in 15 minutes.

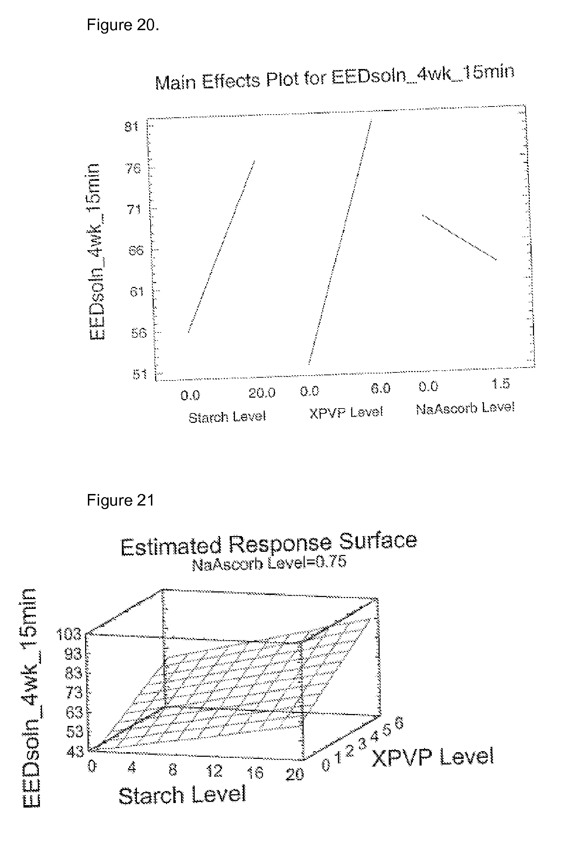

**Figure 20** shows a main effects plot for percent release of esterified estrogens in dissolution tests for 4-week open-dish samples as a function of starch content and crospovidone level, holding sodium ascorbate content at the median level. Samples were stored at 40°C/75%RH conditions. Values for factors are expressed in mg/tablet; esterified estrogens dissolution values are percent released in 15 minutes.

**Figure 21** shows a response plot for percent release of esterified estrogens in dissolution tests for 4-week open-dish samples as a function of starch content and crospovidone level, holding sodium ascorbate content at the median level. Samples were stored at 40°C/75%RH conditions. Values for factors are expressed in mg/tablet; esterified estrogens dissolution values are percent released in 15 minutes.

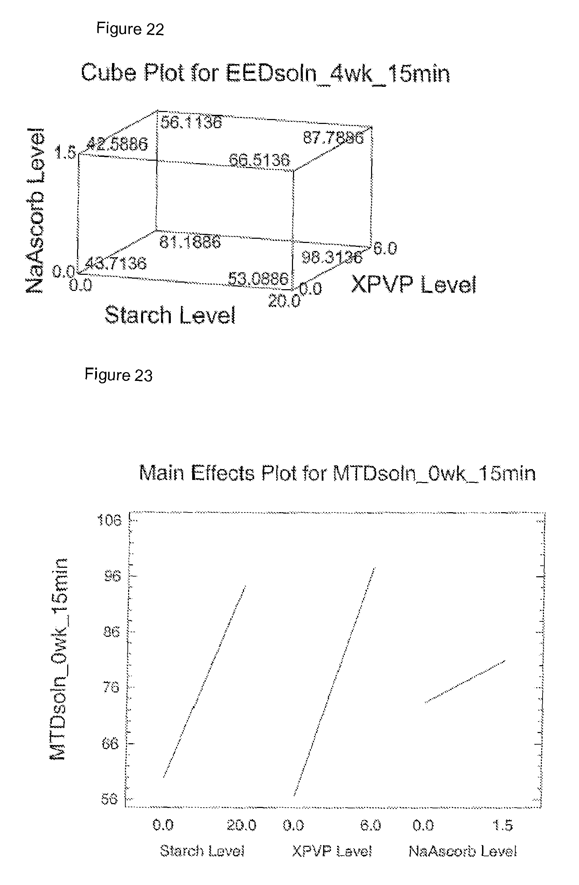

**Figure 22** shows a cube plot for percent release of esterified estrogens in dissolution tests for 4-week open-dish samples as a function of starch, crospovidone and sodium ascorbate levels. Samples stored at 40°C/75%RH conditions. Values for factors are expressed in mg/tablet; esterified estrogens dissolution values are percent released in 15 minutes.

**Figure 23** shows a main efects plot for percent release of methyltestosterone in dissolution tests for time 0 open-dish samples as a function of starch content and crospovidone level, holding sodium ascorbate content at the median level. Samples stored at 40°C/75%RH conditions. Values for factors are expressed in mg/tablet; methyltestosterone dissolution values are percent released in 15 minutes.

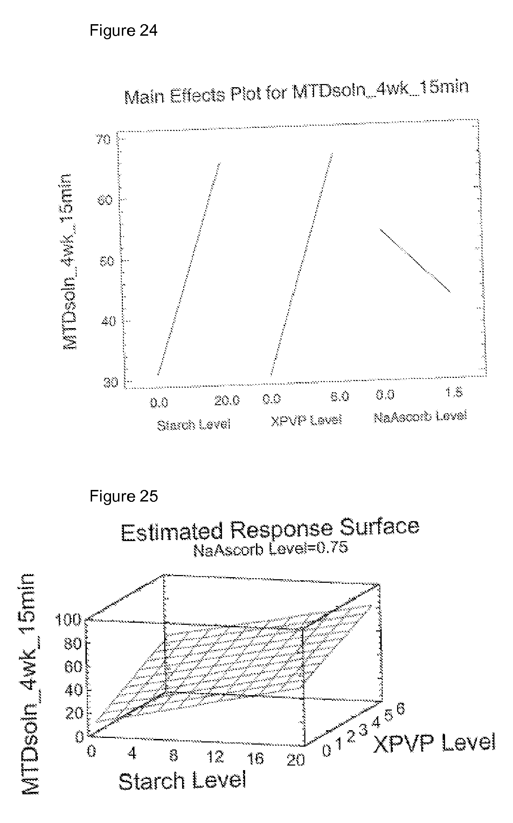

**Figure 24** shows a main effects plot for percent release of methyltestosterone in dissolution tests for 4-week open-dish samples as a function of starch content and crospovidone level, holding sodium ascorbate content at the median level. Samples stored at 40°C/75%RH conditions. Values for factors are expressed in mg/tablet; methyltestosterone dissolution values are percent released in 15 minutes.

**Figure 25** shows a response plot for percent release of methyltestosterone in dissolution tests for 4-week open-dish samples as a function of starch content and crospovidone level, holding sodium ascorbate content at the median level. Samples stored at 40°C/75%RH conditions. Values for factors are expressed in mg/tablet; methyltestosterone dissolution values are percent released in 15 minutes.

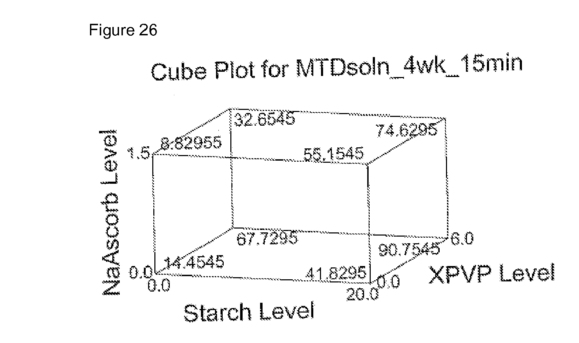

**Figure 26** shows a cube plot for percent release of methyltestosterone in dissolution tests for 4-week open-dish samples as a function of starch, crospovidone and sodium ascorbate levels. Samples were stored at 40°C/75%RH conditions. Values for factors are expressed in mg/tablet; methyltestosterone dissolution values are percent released in 15 minutes.

### DETAILED DESCRIPTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any way. Embodiments illustrated under any heading may be combined with embodiments illustrated under any other heading.

It has been discovered that a pharmaceutical composition comprising at least one esterified estrogen, methyltestosterone and one or more pharmaceutical excipients can provide a superior disintegration profile than known compositions and methods.

It is therefore provided herein a pharmaceutical composition comprising at least one esterified estrogen, methyltestosterone and one or more pharmaceutical excipients having a dissolution of at least 55% in 10 minutes, 65% in 20 minutes, and 75% in 30 minutes. The dissolution profiles for Estrone Sulfate (ESs) and Methyltestosterone (MT) for the invention verses current commercial product are illustrated in Figures 1 and 2, respectively.

It is further provided herein a pharmaceutical composition comprising at least one esterified estrogen, methyltestosterone and one or more pharmaceutical excipients having a disintegration time of about 100 seconds to about 500 seconds for a sample that is about 0 weeks old.

It is further provided herein a pharmaceutical composition comprising at least one esterified estrogen, methyltestosterone and one or more pharmaceutical excipients having a disintegration time of about 300 seconds to about 1200 seconds for a sample that is about 4 weeks old.

It is also provided herein a pharmaceutical composition comprising at least one esterified estrogen, methyltestosterone and a moisture scavenging agent.

Methods of using such compositions for the treatment or prevention of menopause and the symptoms thereof such as vasomotor symptoms, myocardial infarction, stroke, invasive breast cancer, pulmonary emboli, and deep vein thrombosis.

### Esterified Estrogens and Methyltestosterone

In one embodiment, compositions of the present disclosure comprise esterified estrogens (*e.g*. esterified estrogens, USP), including pharmaceutically acceptable salt forms thereof. In another embodiment, compositions of the disclosure comprise methyltestosterone, including pharmaceutically acceptable salt forms thereof. In yet a further embodiment, compositions of the present disclosure comprise combinations of esterified estrogens and methyltestosterone.

Esterified estrogens, USP is a mixture of the sodium salts of the sulfate esters of the estrogenic substances, principally estrone, that are of the type excreted by pregnant mares. In one embodiment, esterified estrogens contain about 70% to about 90%, or about 75% to about 85% of sodium estrone sulfate, for example, about 70%, about 71%, about 72%, about 73%, about 74%, about 75%, about 76%, about 77%, about 78%, about 79%, about 80%, about 81 %, about 82%, about 83%, about 84%, about 85%, about 86%, about 87%, about 88%, about 89% or about 90%; and about 4% to about 20%, or about 6% to about 15%, of sodium equilin sulfate for example, about 4%, about 5%, about 6%, about 7%, about 8%, about 9%, about 10%, about 11 %, about 12%, about 13%, about 14%, about 15%, about 16%, about 17%, about 18%, about 19% or about 20%. In another embodiment, esterified estrogens comprise sodium estrone sulfate and sodium equilin sulfate in such proportion that the total of these two components is about 85%, about 88% or about 90%.

In another embodiment, a composition of the disclosure comprises about 0.1 mg to about 1 mg, about 0.15 mg to about 0.8 mg, about 0.2 to about 0.7 mg, or about 0.3 to about 0.65 mg of esterified estrogens, for example about 0.15 mg, about 0.2 mg, about 0.3 mg, about 0.4 mg, about 0.45 mg or about 0.6 mg of esterified estrogens. In another embodiment, a composition of the disclosure comprises not more than about 0.45 mg of esterified estrogens. In yet a further embodiment, a composition of the disclosure comprises less than 0.45 mg (without being preceded by an approximation) of esterified estrogens.

Methyltestosterone is a white to light yellow crystalline substance of structural Formula I:

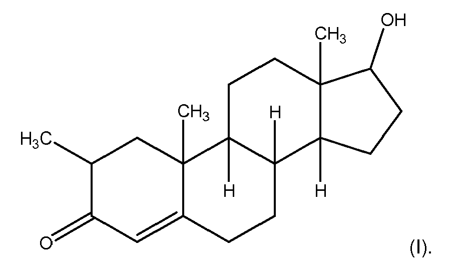

In one embodiment, compositions of the disclosure comprise about 0.1 mg to about 3 mg, about 0.15 mg to about 2 mg, about 0.2 to about 1.5 mg, or about 0.3 to about 1.25 mg of methyltestosterone, for example about 0.15 mg, about 0.3 mg, about 0.6 mg or about 1.25 mg of methyltestosterone.

In another embodiment, a composition of the disclosure comprises esterified estrogens and methyltestosterone in a weight ratio of about 3:1 to about 1:3, about 2:1 to about 1:2, about 1.75:1 to about 1:1.75, about 1.5:1 to about 1:1.5, about 1.33:1 to about 1:1.33, or about 1.25:1 to about 1:1.25, for example about 1:1.

### Moisture Scavenger

In one embodiment, a composition of the disclosure comprises a moisture scavenger. As used herein, a "moisture scavenger" or "moisture scavenging agent" is an agent that performs one or more of the following: absorbs moisture; absorbs water; has a greater affinity for water than esterified estrogens, USP; prevents or inhibits water from reacting with estrogens or testosterones; exhibits high capillary activity; exhibits pronounced hydration capacity; enhances dissolution; enhances the solubility of therapeutic agents; is mostly insoluble in water; is mostly insoluble in organic solvents; or when administered with esterified estrogens obtains a dissolution profile substantially shown in Figures 1 and 2.

Non-limiting examples of suitable moisture scavengers include cross-linked polyvinylpyrolidone (CL-PVP or crospovidone USP/NF including Polyplasdone®), starches, including pregelatinized starch (e.g., Starch 1500®), directly compressible starch, hydrolyzed starches (e.g., Celutab^{™} and Emdex^{™}) sodium starch glycolate (e.g., Explotab^{™} of PenWest) and pregelatinized corn starches (e.g., National^{™} 1551, National^{™} 1550, and Starch 1500™), celluloses and cellulosic materials, such as purified cellulose, microcrystalline cellulose, methylcellulose, carboxymethylcellulose and sodium carboxymethylcellulose, food grade sources of - and amorphous cellulose (e.g., Rexcel^{™}) and powdered cellulose, silica, tribasic calcium phosphate, sodium carboxymethylcellulose (sodium CMC), croscarmellose sodium (e.g., Ac-Di-Sol™ of FMC), and the like. In one embodiment, the moisture scavenger is present in a composition of the disclosure in an amount of about 0.5% to about 15%, about 1 % to about 10%, about 2% to about 8%, or about 3% to about 7%, by weight, for example about 1%, about 2%, about 3%, about 4%, about 5%, about 6%, about 7%, about 8%, about 9%, about 10%, about 11%, about 12% about 13%, about 14% or about 15%, by weight. In another embodiment, the moisture scavenger comprises CR-PVP and is present in an amount of about 0% to about 6%.

For example, by way of illustration and not limitation, in one embodiment, a moisture scavenger is present in a composition of the disclosure in an amount of about 3%, and an alkalizing agent is present in a composition in an amount of about 2%. Further embodiments of the disclosure include compositions where the weight ratio of moisture scavenger to alkalizing agent is about 1.5 (3/2), not more than about 1.5, or not less than about 1.5. Furthermore, 1.5 can be the upper or lower limit in a range formed with any other ratio derivable herein. Accordingly, the skilled person will appreciate that many such ratios, ranges, and ranges of ratios can be unambiguously derived from the data and numbers presented herein and all represent embodiments of the present disclosure.

The foregoing excipients can have multiple roles. For example, crospovidone can serve as a moisture scavenger, as found in the present invention, or it can serve as a disintegrant as known in the art. The classification of moisture scavenging agents above is not to be construed as limiting in any manner. Moisture scavenging agents categorized in any way may operate under various different categories but may still be appreciated by their function as moisture scavengers by one of ordinary skill in the art. The foregoing lists of suitable moisture scavengers are meant to be illustrative and not exhaustive as a person of ordinary skill in the art would recognize that there are many other suitable moisture scavenging agents which could be created.

### Alkalizing Agent

In another embodiment, compositions of the disclosure comprise one or more pharmaceutically acceptable alkalizing agents. The term "alkalizing agent" herein refers to pharmaceutically acceptable agents possessing pharmacological activity as a weak or strong base.

In one embodiment, alkalizing agents useful in accordance with the present disclosure comprise a salt of an alkali (Group IA) earth metal or an akaline (Group IIA; *e.g*. beryllium, magnesium, calcium, strontium, barium, radium) earth metal. Illustrative salts include bicarbonates, carbonates, phosphates, citrates, borates, acetates, phthalates, tartrate, succinates, etc. Additional classes of alkalizing agents useful in accordance with the present disclosure include an aluminum alkalizing agent, a bismuth alkalizing agent, a calcium alkalizing agent, a sodium alkalizing agent, or a magnesium alkalizing agent.

Non-limiting examples of suitable alkalizing agents include aluminum, magnesium hydroxide, aluminum hydroxide/magnesium hydroxide co-precipitate, aluminum hydroxide/sodium bicarbonate co-precipitate, aluminum glycinate, bismuth subcitrate, bismuth subcarbonate, bismuth subsalicylate, calcium acetate, calcium bicarbonate, calcium borate, calcium carbonate, calcium citrate, calcium gluconate, calcium glycerophosphate, calcium hydroxide, calcium lactate, calcium phthalate, calcium phosphate, calcium succinate, calcium tartrate, dibasic sodium phosphate, dipotassium hydrogen phosphate, dipotassium phosphate, disodium hydrogen phosphate, disodium succinate, dry aluminum hydroxide gel, L-arginine, magnesium acetate, magnesium aluminate, magnesium borate, magnesium bicarbonate, magnesium carbonate, magnesium citrate, magnesium gluconate, magnesium hydroxide, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, and trometarnol. (Based in part upon the list provided in The Merck Index, Merck & Co. Rahway, N.J. (2001)). Furthermore, combinations or mixtures of the above mentioned alkalizing agents can be used in compositions described herein.

In various other embodiments of the present disclosure, the alkalizing agent is present in a composition of the disclosure in a total amount of about 0.1 to about 5%, about 0.3 to about 3%, about 0.5% to about 2%, or about 0.75% to about 1.5%, by weight, for example about 0.2%, about 0.4%, about 0.6%, about 0.9%, about 1%, about 1.2%, about 1.4%, about 1.6%, about 1.8%, about 2%, about 2.4%, about 2.6%, about 2.8%, about 3%, abut 3.2%, about 3.4% about 3.6%, about 3.8%, about 4%, about 4.2%, about 4.4%, about 4.6%, about 4.8% or about 5%, by weight.

The foregoing lists of suitable alkalizing agents are meant to be illustrative and not exhaustive as a person of ordinary skill in the art would recognize that there are many other suitable alkalizing agents which could be created.

### Pharmaceutical Excipients

Various embodiments can, if desired, include one or more pharmaceutically acceptable excipients. The term "pharmaceutically acceptable excipient" herein means any substance, used as a carrier or vehicle for delivery of a therapeutic agent to a subject or added to a pharmaceutical composition to improve its handling or storage properties or to permit or facilitate formation of a unit dose of the composition. Excipients include, by way of illustration and not limitation, diluents, disintegrants, binding agents, adhesives, wetting agents, lubricants, glidants, surface modifying agents, substances added to mask or counteract a disagreeable taste or odor, flavors, dyes, fragrances, and substances added to improve appearance of the composition. Any such excipients can be used in any dosage forms of according to the present disclosure, including liquid, solid or semi-solid dosage forms. Excipients optionally employed in various embodiments can be solids, semi-solids, liquids or combinations thereof. Compositions of the disclosure including excipients can be prepared by various pharmaceutical techniques such as admixing an excipient with a drug or therapeutic agent.

Compositions of the disclosure optionally comprise one or more pharmaceutically acceptable diluents as excipients. Suitable diluents illustratively include, either individually or in combination, lactose, including anhydrous lactose and lactose monohydrate; starches, including directly compressible starch and hydrolyzed starches (e.g., Celutab^{™} and Emdex^{™}); mannitol; sorbitol; xylitol; dextrose (e.g., Cerelose^{™} 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of - and amorphous cellulose (e.g., Rexcel^{™}) and powdered cellulose; calcium carbonate; glycine; bentonite; polyvinylpyrrolidone; and the like. Such diluents, if present, constitute in total about 5% to about 99%, about 10% to about 85%, or about 20% to about 80%, of the total weight of the composition. In various embodiments, the diluent or diluents selected may exhibit suitable flow properties and, where tablets are desired, compressibility.

The use of extragranular microcrystalline cellulose (that is, microcrystalline cellulose added to a wet granulated composition after a drying step) can be used to alter or control hardness (for tablets) and/or disintegration time.

Compositions of the disclosure optionally comprise one or more pharmaceutically acceptable disintegrants as excipients. Suitable disintegrants include, either individually or in combination, starches, including sodium starch glycolate (e.g., Explotab^{™} of PenWest) and pregelatinized corn starches (e.g., National^{™} 1551, National^{™} 1550, and Starch 1500™), clays (e.g., Veegum^{™} HV), celluloses such as purified cellulose, microcrystalline cellulose, methylcellulose, carboxymethylcellulose and sodium carboxymethylcellulose, croscarmellose sodium (e.g., Ac-Di-Sol™ of FMC), alginates, crospovidone, and gums such as agar, guar, xanthan, locust bean, karaya, pectin and tragacanth gums.

Disintegrants may be added at any suitable step during the preparation of the composition, particularly prior to a granulation step or during a lubrication step prior to compression. Such disintegrants, if present, typically comprise in total about 0.2% to about 30%, about 0.2% to about 10%, or about 0.2% to about 5%, of the total weight of the composition.

In one embodiment, crosslinked polyvinylpyrrolidone (crospovidone USP/NF) is an optional disintegrant for tablet or capsule disintegration, and, if present, may optionally constitute about 1% to about 5% of the total weight of the composition. In another embodiment, chitin is an optional disintegrant for tablet or capsule disintegration. In still another embodiment, chitosan is an optional disintegrant for tablet or capsule disintegration. In still another embodiment, carboxymethyl cellulose (sodium CMC) is an optional disintegrant for tablet or capsule disintegration. In another embodiment, croscarmellose sodium is a disintegrant for tablet or capsule disintegration, and, if present, may optionally constitute about 0.2% to about 10%, about 0.2% to about 7%, or about 0.2% to about 5%, of the total weight of the composition.

Compositions of the disclosure optionally comprise one or more antioxidants. Illustrative antioxidants include sodium ascorbate, vitamin E (tocopherol) and vitamin E esters, ascorbyl palmitate, phosphatidylcholine, cysteine hydrochloride anhydrous, guaiac extract, ethyl maltol, erythorbic acid, etidronic acid, propyl gallate, methyl and ethyl gallate, gallic acid, cyclodextrin, hydroxypropyl-alpha-cyclodextrin, butylated hydroxytoluene, butylated hydroxyanisole, methyl and ethyl alpha as well as beta cyclodextrins,sodium and potassium bisulfite,sodium and potassium metabisulfite, sodium hypophosphate, sodium thiosulfate anhydrous, sodium formaldehyde sulfoxylate, sucrose, hydroquinone monomethyl ether, zinc glycinate and others. One or more antioxidants, if present, are typically present in a composition of the disclosure in an amount of about 0.001% to about 5%, about 0.005% to about 2.5%, or about 0.01 % to about 1 %, by weight.

Compositions of the disclosure optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, either individually or in combination, acacia; tragacanth; sucrose; gelatin; glucose; starches such as, but not limited to, pregelatinized starches (e.g., National^{™} 1511 and National^{™} 1500); celluloses such as, but not limited to, methylcellulose (*e.g*. Methocel^{™}) and carmellose sodium (e.g., Tylose^{™}); alginic acid and salts of alginic acid; magnesium aluminum silicate; PEG; guar gum; polysaccharide acids; bentonites; povidone, for example povidone K-15, K-30 and K-29/32; polymethacrylates; HPMC; hydroxypropylcellulose (e.g., Klucel™); and ethylcellulose (e.g., Ethocel™). Such binding agents and/or adhesives, if present, constitute in total about 0.5% to about 25%, about 0.75% to about 15%, or about 1 % to about 10%, of the total weight of the composition.

Compositions of the disclosure optionally comprise one or more pharmaceutically acceptable wetting agents as excipients. Non-limiting examples of surfactants that can be used as wetting agents in compositions of the disclosure include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (e.g., Labrasol^{™} of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (e.g., Tween^{™} 80 of ICI), propylene glycol fatty acid esters, for example propylene glycol laurate (e.g., Lauroglycol^{™} of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, about 0.4% to about 10%, or about 0.5% to about 5%, of the total weight of the composition.

Compositions of the disclosure optionally comprise one or more pharmaceutically acceptable lubricants (including anti-adherents and/or glidants) as excipients. Suitable lubricants include, either individually or in combination, glyceryl behenate (e.g., Compritol^{™} 888); stearic acid and salts thereof, including magnesium (magnesium stearate), calcium and sodium stearates; hydrogenated vegetable oils (e.g., Sterotex™); colloidal silica; talc; waxes; boric acid; sodium benzoate; sodium acetate; sodium fumarate; sodium chloride; DL-leucine; PEG (e.g., Carbowax^{™} 4000 and Carbowax^{™} 6000); sodium oleate; sodium lauryl sulfate; and magnesium lauryl sulfate. Such lubricants, if present, constitute in total about 0.1% to about 10%, about 0.2% to about 8%, or about 0.25% to about 5%, of the total weight of the composition.

Suitable anti-adherents include talc, cornstarch, DL-leucine, sodium lauryl sulfate and metallic stearates. Talc is a anti-adherent or glidant used, for example, to reduce formulation sticking to equipment surfaces and also to reduce static in the blend. Talc, if present, constitutes about 0.1 % to about 10%, about 0.25% to about 5%, or about 0.5% to about 2%, of the total weight of the composition. Glidants can be used to promote powder flow of a solid formulation. Suitable glidants include colloidal silicon dioxide, starch, talc, tribasic calcium phosphate, powdered cellulose and magnesium trisilicate.

Glidants can be used to promote powder flow of a solid formulation. Suitable glidants include, without limitation, colloidal silicon dioxide, starch, talc, tribasic calcium phosphate, powdered cellulose and magnesium trisilicate.

Compositions of the present disclosure can comprise one or more flavoring agents, sweetening agents, and/or colorants. Flavoring agents useful in the present disclosure include, without limitation, acacia syrup, alitame, anise, apple, aspartame, banana, Bavarian cream, berry, black currant, butter, butter pecan, butterscotch, calcium citrate, camphor, caramel, cherry, cherry cream, chocolate, cinnamon, citrus, citrus punch, citrus cream, cocoa, coffee, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, MagnaSweet®, maltol, mannitol, maple, menthol, mint, mint cream, mixed berry, nut, orange, peanut butter, pear, peppermint, peppermint cream, Prosweet® Powder, raspberry, root beer, rum, saccharin, safrole, sorbitol, spearmint, spearmint cream, strawberry, strawberry cream, stevia, sucralose, sucrose, Swiss cream, tagatose, tangerine, thaumatin, tutti fruitti, vanilla, walnut, watermelon, wild cherry, wintergreen, xylitol, and combinations thereof, for example, anise-menthol, cherry-anise, cinnamon-orange, cherry-cinnamon, chocolate-mint, honey-lemon, lemon-lime, lemon-mint, menthol-eucalyptus, orange-cream, vanilla-mint, etc.

Sweetening agents that can be used in the present disclosure include, for example, acesulfame potassium (acesulfame K), alitame, aspartame, cyclamate, cylamate, dextrose, isomalt, MagnaSweet®, maltitol, mannitol, neohesperidine DC, neotame, Prosweet® Powder, saccharin, sorbitol, stevia, sucralose, sucrose, tagatose, thaumatin, xylitol, and the like.

Flavoring agents, sweetening agents, and/or colorants can be present in compositions of the disclosure in any suitable amount, for example about 0.01% to about 10%, about 0.1% to about 8%, or about 1 % to about 5%, by weight.

In one embodiment, a composition of the disclosure is substantially free of sucrose. In another embodiment, a composition of the disclosure is free of a sugar coating.

The foregoing excipients can have multiple roles as is known in the art. For example, crospovidone can serve as a moisture scavenger as well as an excipient; starch can serve as a filler as well as a disintegrant. The classification of excipients above is not to be construed as limiting in any manner. Excipients categorized in any way may also operate under various different categories of excipients as will be readily appreciated by one of ordinary skill in the art.

### Dissolution and Disintegration

Dissolution is measured according to USP chapter <711>. USP apparatus 1 (Basket Apparatus) is used for the determination. The dissolution media is 10 % simulated intestinal fluid and the rotation speed is 50 rpm. The invention is an immediate release product as demonstrated by the dissolution profiles presented in Figures 1 and 2.

It is further provided herein a pharmaceutical composition comprising esterified estrogen, methyltestosterone and one or more pharmaceutical excipients having a disintegration time of about 100 seconds to about 500 seconds for a sample that is about 0 weeks old, for example, about 100 seconds, about 110 seconds, about 120 seconds, about 130 seconds, about 140 seconds, about 150 seconds, about 160 seconds, about 170 seconds, about 180 seconds, about 190 seconds, about 200 seconds, about 210 seconds, about 220 seconds, about 230 seconds, about 240 seconds, about 250 seconds, about 260 seconds, about 270 seconds, about 280 seconds, about 290 seconds, about 300 seconds, about 310 seconds, about 320 seconds, about 330 seconds, about 340 seconds, about 350 seconds, about 360 seconds, about 370 seconds, about 380 seconds, about 390 seconds, about 400 seconds, about 410 seconds, about 420 seconds, about 430 seconds, about 435 seconds, about 440 seconds, about 445 seconds, about 450 seconds, about 460 seconds, about 470 seconds, about 480 seconds, about 490 seconds, or about 500 seconds.

In various embodiments, disclosed herein is a pharmaceutical composition comprising esterified estrogen, methyltestosterone and one or more pharmaceutical excipients having a disintegration time of about 300 seconds to about 1200 seconds for a sample that is about 4 weeks old, for example about 300 seconds, about 310 seconds, about 320 seconds, about 330 seconds, about 340 seconds, about 350 seconds, about 360 seconds, about 370 seconds, about 380 seconds, about 390 seconds, about 400 seconds, about 410 seconds, about 420 seconds, about 430 seconds, about 435 seconds, about 440 seconds, about 445 seconds, about 450 seconds, about 460 seconds, about 470 seconds, about 480 seconds, about 490 seconds, about 500 seconds, about 510 seconds, about 515 seconds, about 520 seconds, about 530 seconds, about 540 seconds, about 550 seconds, 560 seconds, about 565 seconds, about 570 seconds, about 580 seconds, about 590 seconds, about 600 seconds, about 610 seconds, about 620 seconds, about 630 seconds, about 640 seconds, about 650 seconds, about 660 seconds, about 670 seconds, about 680 seconds, about 690 seconds, about 700 seconds, about 710 seconds, about 720 seconds, about 730 seconds, about 740 seconds, about 750 seconds, about 760 seconds, about 770 seconds, about 780 seconds, about 790 seconds, about 800 seconds, about 810 seconds, about 820 seconds, about 830 seconds, about 840 seconds, about 850 seconds, about 860 seconds, about 870 seconds, about 880 seconds, about 890 seconds, about 900 seconds, about 910 seconds, about 920 seconds, about 930 seconds, about 940 seconds, about 950 seconds, about 960 seconds, about 970 seconds, about 980 seconds, about 990 seconds, about 1000, about 1010, about 1020, about 1030, about 1040, about 1050, about 1060, about 1070, about 1080, about 1090 seconds, about 1100 seconds, about 1110 seconds, about 1120 seconds, about 1130 seconds, about 1140 seconds, about 1150 seconds, about 1160 seconds, about 1170 seconds, about 1180 seconds, about 1185 seconds, or about 1110 seconds.

### Storage Stability

In one embodiment, compositions of the disclosure, upon storage in an open or closed container maintained at 40 °C/75% RH, ambient conditions, refrigerated (*e.g*. about 5°C - 10 °C) temperature, or freezing temperature for a period of about 1 week, about 2 weeks, about 3 weeks, or about 1, about 2, about 3, about 4, about 5, about 6, about 7, about 8, about 9, about 10, about 11, or about 12 months, contain about 90%, about 92.5%, about 95%, about 97.5%, or about 99% of the originally present sodium equilin sulfate (a component of esterified estrogens) in non-degraded form.

In another embodiment, compositions of the disclosure, upon storage in an open or closed container maintained at 40 °C/75% RH, ambient conditions, refrigerated (*e.g*. about 5 - 10 °C) temperature, or freezing temperature for a period of about 1 week, about 2 weeks, about 3 weeks, or about 1, about 2, about 3, about 4, about 5, about 6, about 7, about 8, about 9, about 10, about 11, or about 12 months, contain about 90%, about 92.5%, about 95%, about 97.5%, or about 99% of the originally present sodium estrone sulfate (a component of esterified estrogens) in non-degraded form.

In one embodiment, the above stability functionality can be achieved by selecting, by type and/or amount, proper moisture scavengers and optionally one or more additional pharmaceutically acceptable excipients (*e.g*. antioxidants). Such moisture scavengers and optional one or more additional pharmaceutically acceptable excipients can be combined in various ratios and amounts, through routine experimentation, to prepare pharmaceutical compositions meeting the above stability criteria. In another embodiment, the above stability functionality can be achieved by coating the orally deliverable dosage unit with a moisture barrier such as polyvinylalcohol, PVA and Xanthan gum and/or PVA with hydroxypropylmethylcellulose.

In another embodiment, the present disclosure provides a method for stabilizing esterified estrogens comprising co-formulating the esterified estrogens with an agent that absorbs water. In a related embodiment, the agent that absorbs water is CL-PVP as is described herein.

### Pharmaceutical Dosage Forms

In one embodiment, compositions of the disclosure are in the form of an orally deliverable dosage unit. The terms "orally deliverable" or "oral administration" herein includes any form of delivery of a therapeutic agent or a composition thereof to a subject wherein the agent or composition is placed in the mouth of the subject, whether or not the agent or composition is swallowed. Thus "oral administration" includes buccal and sublingual as well as esophageal administration.

Compositions of the present disclosure can be formulated as solid, liquid or semi-solid dosage forms. In one embodiment, such compositions are in the form of discrete dose units or dosage units. The terms "dose unit" and/or "dosage unit" herein refer to a portion of a pharmaceutical composition that contains an amount of a therapeutic agent suitable for a single administration to provide a therapeutic effect. Such dosage units may be administered one to a small plurality (*i.e*. 1 to about 4) times per day, or as many times as needed to elicit a therapeutic response. A particular dosage form can be selected to accommodate any desired frequency of administration to achieve a specified daily dose. Typically one dose unit, or a small plurality (*i.e.* up to about 4) of dose units, provides a sufficient amount of the active drug to result in the desired response or effect.

In various embodiments, compositions of the disclosure are in the form of solid dosage forms or dosage units. Non-limiting examples of suitable solid dosage forms include tablets (*e.g*., immediate release tablets, suspension tablets, bite suspension tablets, rapid dispersion tablets, chewable tablets, effervescent tablets, bilayer tablets, *etc*), caplets, capsules (*e.g*. a soft or a hard gelatin capsule), powder (*e.g*. a packaged powder, a dispensable powder or an effervescent powder), lozenges, sachets, cachets, troches, pellets, granules, microgranules, encapsulated microgranules, powder aerosol formulations, or any other solid dosage form reasonably adapted for oral administration.

Tablets can be prepared according to any of the many relevant, well known pharmacy techniques. In one embodiment, tablets or other solid dosage forms can be prepared by processes that employ one or a combination of methods including, without limitation, (1) dry mixing, (2) direct compression, (3) milling, (4) dry or non-aqueous granulation, (5) wet granulation, or (6) fusion.

The individual steps in the wet granulation process of tablet preparation typically include milling and sieving of the ingredients, dry powder mixing, wet massing, granulation and final grinding. Dry granulation involves compressing a powder mixture into a rough tablet or "slug" on a heavy-duty rotary tablet press. The slugs are then broken up into granular particles by a grinding operation, usually by passage through an oscillating granulator. The individual steps include mixing of the powders, compressing (slugging) and grinding (slug reduction or granulation). Typically, no wet binder or moisture is involved in any of the steps.

In another embodiment, solid dosage forms can be prepared by mixing esterified estrogens with methyltestosterone as described herein above and, if desired, with one or more optional pharmaceutical excipients to form a substantially homogeneous preformulation blend. The preformulation blend can then be subdivided and optionally further processed (*e.g*. compressed, encapsulated, packaged, dispersed, granulated, *etc*.) into any desired dosage forms.

Compressed tablets can be prepared by compacting a powder or granulation composition of the disclosure. The term "compressed tablet" generally refers to a plain, uncoated tablet suitable for oral ingestion, prepared by a single compression or by pre-compaction tapping followed by a final compression. Tablets of the present disclosure may be coated or otherwise compounded to provide a dosage form affording the advantage of improved handling or storage characteristics. In one embodiment, any such coating will be selected so as to not substantially delay onset of therapeutic effect of a composition of the disclosure upon administration to a subject. The term "suspension tablet" as used herein refers to a compressed tablet that rapidly disintegrates after placement in water.

In embodiments of the present disclosure, immediate release tablets each may comprise (% w/w) of the following according to the exemplary formulations in Table 1 below:

**Table 1**

| | 1 | 2 | 3 | 4 | 5 | 6 | 7 | 8 | 9 | 10 |
|---|---|---|---|---|---|---|---|---|---|---|
| Esterified estrogens USP | 0.15% | 0.30% | 0.30% | 0.60% | 0.15% | 0.30% | 0.60% | 0.30% | 0.60% | 0.60% |
| Methyltestostero ne USP | 0.15% | 0.15% | 0.30% | 0.30% | 0.60% | 0.60% | 0.60% | 1.20% | 1.0% | 0% |
| Anti-oxidant | 0% | 0.75% | 1.5% | 0% | 0.75% | 1.5% | 0% | 0.75% | 1.5% | 1.5% |
| Moisture scavenger | 6% | 3% | 3% | 1.5% | 6% | 3% | 3% | 6% | 3% | 6% |
| Other excipients | qs to 100% w/w | qs to 100% w/w | qs to 100% w/w | qs to 100% w/w | qs to 100% w/w | qs to 100% w/w | qs to 100% w/w | qs to 100% w/w | qs to 100% w/w | qs to 100% w/w |

While not intending to be bound by theory, the inventor believes that the moisture scavenger (e.g., crospovidone) unexpectedly and synergistically acts with the other components to stabilize the formulation.

In making the formulations listed in Table 2 and the Examples herein, the sugar binder/diluent, esterified estrogens triturate, methyltestosterone, alkalizing agent, anti-oxidant, super-disintegrant/moisture scavenging agent and cellulosic binder/diluent/moisture scavenger are blended in a suitable sized blender equipped with high intensity mixer for an optimized time-period (for example, 5 to 15 minutes). The pre-sifted glidant is next blended with the powders; and finally lubricated with a suitable lubricant. The final blend is compressed on a fast-speed rotary press to the desired weight, thickness and hardness. The core-tablets are then film-coated with a moisture barrier.

The formulations of the present disclosures showed marked improvement in physical characteristics, *i.e*. disintegration and dissolution of the tablets when the super-disintegrant crospovidone was introduced. Crospovidone also unexpectedly enhanced stability. The introduction of an anti-oxidant further improved stability of the formulation. Blend uniformity and granulation flow in the press' hopper was markedly impacted by replacing the sugar binder/diluent with a spray-dried grade and the cellulosic binder/diluent/moisture scavenger by a denser and larger particle size grade with low moisture content. The film-coating with a moisture barrier further enhanced its stability.

In various embodiments, formulations of the present disclosures comprise about 3% to about 17.2% by weight esterified estrogens, USP triturate. For example, about 3%, about 3.25%, about 3.5%, about 3.75%, about 4%, about 4.25%, about 4.5%, about 4.75%, about 5%, about 5.25%, about 5.5%, about 5.75%, about 6%, about 7%, about 8%, about 9%, about 10%, about 11 %, about 12%, about 13%, about 14%, about 15%, about 16%, or about 17%.

In one embodiment, formulations of the present disclosures comprise about 0.15mg to about 0.6mg by dose of esterified estrogens USP, for example about 0.15mg, about 0.20mg, about 0.25mg, about 0.30mg, about 0.35mg, about 0.40mg, about 0.45mg, about 0.50mg, about 0.55mg, or about 0.60mg.

In one embodiment, formulations of the present disclosures comprise about 0.15% to about 1.2% by weight of methyltestosterone USP, for example about 0.15%, about 0.20%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 1.0%, about 1.1 %, or about 1.2%.

In one embodiment, formulations of the present disclosures comprise about 0.15mg to about 1.2mg by weight of methyltestosterone USP, for example about 0.15mg , about 0.20mg , about 0.30mg , about 0.35mg , about 0.40mg , about 0.45mg , about 0.50mg , about 0.55mg , about 0.60mg , about 0.65mg , about 0.70mg , about 0.75mg , about 0.80mg , about 0.85mg , about 0.90mg , about 1.0mg , about 1.1mg , or about 1.2mg .

In another embodiment, formulations of the present disclosure comprise about 50% to about 90% by weight of anhydrous lactose, NF, for example about 50%, about 55%, about 60%, about 65%, about 70%, about 75%, about 80%, about 85%, or about 90%.

In another embodiment, formulations of the present disclosure comprise about 50% to about 90% by weight of lactose monohydrate (crystalline or spray dried), NF, for example about 50%, about 55%, about 60%, about 65%, about 70%, about 75%, about 80%, about 85%, or about 90%.

In another embodiment, formulations of the present disclosure comprise about 8% to about 25% by weight of microcrystalline cellulose, NF, for example, about 8%, about 8.1%, about 8.2%, about 8.3%, about 8.4%, about 8.5%, about 8.6%, about 8.7%, about 8.8%, about 8.9%, about 9%, about 9.1%, about 9.2, about 9.3%, about 9.4%, about 9.5%, about 9.6%, about 9.7%, about 9.8%, about 9.9%, about 10%, about 12.5%, about 15%, about 17.5%, about 20%, about 22.5%, or about 25%.

Another embodiment comprises about 0% to about 5%, by weight of sodium bicarbonate, USP, for example, about 0%, 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%. about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, or about 5%.

Still another embodiment comprises about 0% to about 25%, by weight of partially pregelatinized starch, for example. about 0%, about 1%, about 2%, about 3%, about 4%, about 5%, about 6%, about 7%, about 8%, about 9%, about 10%, about 11 %, about 12%, about 13%, about 14%, about 15%, about 16%, about 17%, about 18%, about 19%, about 20%, about 21 %, about 22%, about 23%, about 24% or about 25%.

Another embodiment comprises about 0% to about 15%, by weight of crospovidone NF, for example. about 0%, about 1%, about 2%, about 3%, about 4%, about 5%, about 6%, about 7%, about 8%, about 9%, about 10%, about 11 %, about 12%, about 13%, about 14%, or about 15%.

Yet another embodiment comprises about 0% to about 5%, by weight of sodium ascorbate, USP, for example, about 0%, about 0.05%, about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, about 0.8%, about 0.85%, about 0.9%, about 0.95%, about 1%, about 1.05%, about 1.1%, about 1.15%, about 1.2%, about 1.25%, about 1.3%, about 1.35%, about 1.4%, about 1.45%, about 1.5%, about 1.55%, about 1.6%, about 1.65%, about 1.7%, about 1.75%, about 1.8%, about 1.85%, about 1.9%, about 1.95%, about 2%, about 2.05%, about 2.1%, about 2.15%, about 2.2%, about 2.25%, about 2.3%, about 2.35%, about 2.4%, about 2.45%, about 2.5%, about 2.55%, about 2.6%, about 2.65%, about 2.7%, about 2.75%, about 2.8%, about 2.85%, about 2.9%, about 2.95%, about 3%, about 3.1 %, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, or about 5%.

Another embodiment comprises about 0% to about 5%, by weight of colloidal silicon dioxide, NF, for example, about 0%, 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%. about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, or about 5%.

Still another embodiment comprises about 0% to about 5%, by weight of magnesium stearate, NF, for example, about 0%, 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%. about 1.5%, about 1.6%, about 1.7%, about 1.8%, about 1.9%, about 2%, about 2.1 %, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, or about 5%.

### Administration

It will be understood that the terms "therapeutically effective amount," "prophylactically effective amount," "effective amount" or "amount effective to treat" as used herein refer to an amount of drug or agent that is sufficient to elicit the required or desired therapeutic and/or prophylactic response, as the particular treatment context may require. In one embodiment, a "therapeutically effective amount" is the lowest amount of drug or agent that is sufficient to elicit the required or desired therapeutic and/or prophylactic response, as the particular treatment context may require.

It will be understood that a therapeutically and/or prophylactically effective amount of a drug for a subject is dependent *inter alia* on the body weight of the subject. A "subject" herein to which a therapeutic agent or composition thereof can be administered includes a human subject of either sex and of any age, and also includes any nonhuman animal, particularly a domestic or companion animal, illustratively a cat, dog or a horse.

Compositions of the disclosure can be used to treat and/or prevent numerous medical conditions. In one embodiment, the compositions of the disclosure are useful in the treatment and/or prevention of vasomotor symptoms (*e.g*. hot flushes, sweats, etc.) associated with menopause. In another embodiment, compositions of the disclosure are useful in treatment and/or prevention of chronic sleep deprivation, mood and behavior changes, sexual dysfunction, and bone loss, which are associated with menopause. In still another embodiment the compositions of the disclosure are useful in the treatment and/or prevention of myocardial infarction, stroke, invasive breast cancer, pulmonary emboli, and deep vein thrombosis.

The term "treatment" in relation to a given disease or disorder, includes, but is not limited to, inhibiting the disease or disorder, for example, arresting the development of the disease or disorder; relieving the disease or disorder, for example, causing regression of the disease or disorder; or relieving a condition caused by or resulting from the disease or disorder, for example, relieving, preventing or treating symptoms of the disease or disorder.

The term "prevention" in relation to a given disease or disorder means: preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

Such compositions of the disclosure can be administered one to a small plurality of times per day. The term "small plurality" herein means more than one but less than about 10. For example, a small plurality in the present context could illustratively represent about 2, about 3, about 4, about 5, about 6, about 7, about 8, about 9, or about 10 dosage compartments or any increment within the range of 2 to 10.

In one embodiment, compositions of the disclosure are administered to a subject in an amount sufficient to provide the lowest effective dose of esterified estrogens. The term "lowest effective dose" in the present context means the lowest dose required to elicit the desired therapeutic or prophylactic response. In another embodiment, compositions of the disclosure are administered to a subject in an amount sufficient to provide about 0.1 mg to about 0.45 mg, about 0.1 to about 0.4 mg, or about 0.1 to about 0.35 mg of esterified estrogens per day, for example about 0.2, about 0.22, about 0.24, about 0.26, about 0.28, about 0.3, about 0.32, about 0.34, about 0.36, about 0.38, about 0.4, about 0.42 or about 0.44 mg of esterified estrogens per day.

In another embodiment, esterified estrogens and methyltestosterone are co-administered in a composition of the disclosure and such co-administration reduces the amount of esterified estrogens needed to relieve vasomotor symptoms associated with menopause in the subject being treated (by comparison with esterified estrogens administered without the methyltestosterone).

In this embodiment, the subject being treated may have been taking esterified estrogens (without combination with methyltestosterone) prior to initiating treatment with a composition of the disclosure. In such a case, a composition of the disclosure will be administered to the subject in an amount sufficient to provide the subject with a lower dose of esterified estrogens than they had been taking previously, for example (1) the dose they had been taking immediately prior to initiating treatment with a composition of the disclosure, or (2) the average daily dose of esterified estrogens that they had been taking over the 3 months, 6 months or 12 months prior to initiating treatment with a composition of the disclosure.

In another embodiment, the present disclosure provides a method for reducing the amount of esterified estrogens needed to treat and/or prevent vasomotor symptoms associated with menopause, the method comprising the steps of: preparing or providing a composition as described herein comprising esterified estrogens and methyltestosterone, and administering the composition to a subject in need of treatment for vasomotor symptoms associated with menopause.

Those skilled in the art will readily appreciate that numerous other embodiments, modifications and equivalents are contemplated and encompassed by the disclosure of the present disclosure.

All U.S. Patents and published U.S. patent applications listed herein are hereby incorporated herein by reference in their entirety. All patents, patent applications and publications referenced herein are hereby incorporated by reference herein to the fullest extent allowed under the law.

### EXAMPLES

The following Examples are provided for illustrative purposes only and are not to be interpreted as limiting the scope of the present disclosure in any manner.

### Example 1 - Powder Blends

Several powder blends, A - K, were prepared as shown in Table 2. The powder blends were prepared by introducing into a suitable sized V-Blender equipped with intensifier bar the sugar binder/diluent, esterified estrogens triturate, methyltestosterone, alkalizing agent, antioxidant, super-disintegrant/moisture scavenging agent and cellulosic binder/diluent/moisture scavenger. The powders were blended for 15 minutes with the I-Bar on. The pre-sifted glidant is next blended with the powders for 4 minutes with the I-Bar off.; and finally lubricated with a suitable lubricant by blending for 3 minutes with I-Bar off.. The final blend is compressed on a fast-speed rotary press to the desired weight, thickness and hardness. The core-tablets are then film-coated with a moisture barrier.

**Table 2. Powder Blends A - K**

| | **Weight (g)** | | | | | | | | | | |
|---|---|---|---|---|---|---|---|---|---|---|---|
| **Ingredient** | **A** | **B** | **C** | **D** | **E** | **F** | **G** | **H** | **I** | **J** | **K** |
| Esterified estrogens, USP triturate | 190 | 190 | 190 | 190 | 190 | 190 | 190 | 190 | 190 | 190 | 190 |
| Methyltestosterone, USP | 24 | 24 | 24 | 24 | 24 | 24 | 24 | 24 | 24 | 24 | 24 |
| Anhydrous Lactose, NF | 3313.2 | 3253.2 | 3073.2 | 3013.2 | 2513.2 | 2453.2 | 2273.2 | 2213.2 | 2763.2 | 2763.2 | 2763.2 |
| Microcrystalline cellulose, NF | 392.8 | 392.8 | 392.8 | 392.8 | 392.8 | 392.8 | 392.8 | 392.8 | 392.8 | 392.8 | 392.8 |
| Sodium bicarbonate, USP | 44 | 44 | 44 | 44 | 44 | 44 | 44 | 44 | 44 | 44 | 44 |
| Partially pregelatinized starch | 0 | 0 | 0 | 0 | 800 | 800 | 800 | 800 | 800 | 800 | 800 |
| Crospovidone NF | 0 | 0 | 240 | 240 | 0 | 0 | 240 | 240 | 120 | 120 | 120 |
| Sodium ascorbate USP | 0 | 60 | 0 | 60 | 0 | 60 | 0 | 60 | 30 | 30 | 30 |
| Colloidal silicon dioxide NF | 4 | 4 | 4 | 4 | 4 | 4 | 4 | 4 | 4 | 4 | 4 |
| Magnesium stearate NF | 32 | 32 | 32 | 32 | 32 | 32 | 32 | 32 | 32 | 32 | 32 |

| | | | | | | | | | | | |
|---|---|---|---|---|---|---|---|---|---|---|---|
| The theoretical batch sizes were 4.0Kg. Theoretical weight of tablet: 100.0mg (Range: 92.5mg - 107.5mg) (avg.20) Thickness: To Be Determined (Tentative Range: 0.1200 - 0.1350 inch) Hardness: 6.0Kp (Range: 5.0 Kp - 7.0 Kp) | | | | | | | | | | | |

Two control powder blends, L and M, were compared having the composition as shown in Table 3.

**Table 3. Control Powder Blends L and M**

| | **Weight (mg)** | |
|---|---|---|
| **Ingredient** | **L** | **M** |
| Esterified estrogens, USP triturate | 996 | 190 |
| Methyltestosterone, USP | 24 | 24 |
| Anhydrous Lactose, NF | 1407.2 | 0 |
| Lactose Monohydrate NF | 0 | 2213.2 |
| Microcrystalline cellulose, NF | 392.8 | 392.8 |
| Sodium bicarbonate, USP | 44 | 44 |
| Partially pregelatinized starch | 800 | 800 |
| Crospovidone NF | 240 | 240 |
| Sodium ascorbate USP | 60 | 60 |
| Colloidal silicon dioxide NF | 4 | 4 |
| Magnesium stearate NF | 32 | 32 |

### Example 2 - The Influence of Starch, Crospovidone and Sodium Ascorbate Levels in a New Low-Dose Esterified Estrogens/Methyltestosterone Combination Formulation Tablet Using Experimental Design

A 3-Factor, 2-Level full factorial screening design with centerpoints was used to evaluate the effects of partially pregelatinized starch (Starch 1500®), crospovidone (Polyplasdone®) and sodium ascorbate levels on various characteristics of a new low-dose esterified estrogens/methyltestosterone combination tablet.

The purpose of this study was to investigate the effects of various levels of partially pregelatinized starch, crospovidone and sodium ascorbate on the characteristics of tablets containing a combination of esterified estrogens/methyltestosterone in a controlled stability study under accelerated conditions. These three factors were studied because of their widely accepted properties in direct tableting applications. Partially pregelatinized starch (Starch 1500®) functions as a binder and moisture scavenger; crospovidone is recognized as a superdisintegrant; and, sodium ascorbate serves as an antioxidant in many formulations. Characteristics studied included quantity and rate of sodium equilenin sulfate formation, tablet disintegration time and tablet dissolution rate.

The study was performed using an experimental design to evaluate uncoated tablets of 11 formulations, plus two additional formulations that served as controls, which were stored under accelerated conditions of 40° C/75%RH in open-dish for periods up to four weeks. All formulations attempted produced useable tablets.

While multiple characteristics were evaluated, the primary focus was on the stability of the sodium equilin sulfate component and its degradation product, sodium equilenin sulfate. A goal of the study was minimizing the quantity and rate of formation of sodium equilenin sulfate. Analysis suggested that the minimum rate of formation could be achieved with a formulation containing crospovidone 6.0 mg/tablet, sodium ascorbate 1.5 mg/tablet, and no partially gelatinized starch. In general, crospovidone content was the most significant factor in the study of effects.

The analysis also predicted that the use of this formulation would affect both disintegration time and dissolution release rates for both esterified estrogens and methyltestosterone components. A formulation containing crospovidone 6.0 mg/tablet, sodium ascorbate 1.5 mg/tablet, and no starch is estimated to cause a slight increase in disintegration time from the optimum for 4-Week samples stored at accelerated conditions, which is from approximately 272 seconds to 288 seconds, a 5.9% increase.

The predicted impact of this formulation on dissolution release rate for both active components is more significant. Dissolution rates at the 15-minute test point may decrease from an estimated optimum of approximately 98.3% to 56.1% for this formulation, a 42.9% decrease.

A new formulation is in development for an esterified estrogens (0.15 mg EE)/methyltestosterone (0.6 mg MT) combination tablet product. The tablet is being developed for the purposes of reducing the dosage administered as compared with the current commercial product, reducing the dosage form size, and improving the stability and manufacturing properties of the tablet. To support these activities, a study was initiated to determine the effects varying certain binders, disintegrants and antioxidants in the proposed formulation.

### Materials:

All materials were used as received and complied with current applicable USP/NF standards. Esterified Estrogens, USP was received as a powder triturate (Organics/LaGrange). Methyltestosterone, USP was provided as a micronized powder (Diosynth). Anhydrous lactose NF was received as Anhydrous Lactose Direct Tableting® (Quest International). Lactose monohydrate NF was provided as Pharmatose® DCL-15 (DMV Pharma). Microcrystalline cellulose NF was received as Avicel® PH-103 (FMC). Sodium bicarbonate USP, colloidal silicon dioxide NF, and magnesium stearate NF were used as received from the cGMP manufacturing site for Solvay Pharmaceuticals, Baudette, MN. Partially pregelatinized starch NF was provided as Starch 1500® (Colorcon). Sodium ascorbate USP was used as received (BASF). Associated lot numbers for each material are detailed in the related batch records, Lots 1681-15-1 through -13, on file.

Formulations used for this study are provided in Table 4 below:

**Table 4: Tablet Formulations Used in Low-Dose Esterified Estrogens/Methyltestosterone Experimental Design Study.**

| **Formulations (mg / tablet)** | | | | | | | | | | | | | |
|---|---|---|---|---|---|---|---|---|---|---|---|---|---|
| **Statgraphics Trial No.** | **4** | **9** | **7** | **2** | **3** | **10** | **8** | **5** | **1** | **6** | **11** | **Control (0.625mg)** | **Control (DCL-15)** |
| **Lot Number 1681-15-** | **1** | **2** | **3** | **4** | **5** | **6** | **7** | **8** | **9** | **10** | **11** | **12** | **13** |
| Esterified Estrogens USP Triturate* (3.21 mg/gram) | 4.75 | 4.75 | 4.75 | 4.75 | 4.75 | 4.75 | 4.75 | 4.75 | 4.75 | 4.75 | 4.75 | 24.9 | 4.75 |
| Methyltestosterone USP | 0.60 | 0.60 | 0.60 | 0.60 | 0.60 | 0.60 | 0.60 | 0.60 | 0.60 | 0.60 | 0.60 | 0.60 | 0.60 |
| Anhydrous Lactose NF, DT | 82.83 | 81.33 | 76.83 | 75.33 | 62.83 | 61.33 | 56.84 | 55.33 | 69.08 | 69.08 | 69.08 | 35.18 | 0 |
| Lactose Monohydrate NF (Pharmatose® DCL-15) | 0 | 0 | 0 | 0 | 0 | 0 | 0 | 0 | 0 | 0 | 0 | 0 | 55.33 |
| Microcrystalline cellulose NF (Avicel® PH-103) | 9.82 | 9.82 | 9.82 | 9.82 | 9.82 | 9.82 | 9.82 | 9.82 | 9.82 | 9.82 | 9.82 | 9.82 | 9.82 |
| Sodium Bicarbonate USP | 1.1 | 1.1 | 1.1 | 1.1 | 1.1 | 1.1 | 1.1 | 1.1 | 1.1 | 1.1 | 1.1 | 1.1 | 1.1 |
| Partially Pregelatinized Starch (Starch 1500®) | 0 | 0 | 0 | 0 | 20 | 20 | 20 | 20 | 10 | 10 | 10 | 20 | 20 |
| Crospovidone NF (Polyplasdone® XL-10) | 0 | 0 | 6 | 6 | 0 | 0 | 6 | 6 | 3 | 3 | 3 | 6 | 6 |
| Sodium ascorbate USP | 0 | 1.5 | 0 | 1.5 | 0 | 1.5 | 0 | 1.5 | 0.75 | 0.75 | 0.75 | 1.5 | 1.5 |
| Colloidal silicon dioxide NF | 0.1 | 0.1 | 0.1 | 0.1 | 0.1 | 0.1 | 0.1 | 0.1 | 0.1 | 0.1 | 0.1 | 0.1 | 0.1 |
| Magnesium stearate NF | 0.8 | 0.8 | 0.8 | 0.8 | 0.8 | 0.8 | 0.8 | 0.8 | 0.8 | 0.8 | 0.8 | 0.8 | 0.8 |
| *Contains 5% overage. | | | | | | | | | | | | | |

### Methods:

**Statistical Experimental Design:** A three-factor, two-level full factorial design with three center points was used as a screening design. A range for each factor was predetermined. The design was generated and analyzed using Statgraphics® Plus v. 5.1 (StatPoint, Inc., Herndon, VA) software. The regression equation that is fitted to the data of the screening design is: Y = B₀+B₁X₁+B₂X₂+B₃X₃+B₁₂X₁X₂+B₁₃X₁X₃+B₂₃X₂X₃, where Y is the response, Xᵢ are the factors, and the Bᵢ terms are the coefficients characterizing the main (B₀, B₁, B₂, and B₃) effects and the two-factor interaction (B₁₂, B₁₃, and B₂₃) effects. An Analysis of Variance (ANOVA) was performed for each response employing an error probability of p=0.05. Factors studied were the levels of: (1) partially pregelatinized starch (Starch), 0-20 mg/tablet; (2) crospovidone (XPVP), 0-6 mg/tablet; and, (3) sodium ascorbate (NaAscorb), 0-1.5 mg/tablet. Lactose quantities were allowed to "float" to compensate for the weight differences required to maintain the 100-mg tablet target weight for each formulation. Remaining excipients were held constant in each formulation. Responses determined at specific time points were: (1) quantity (assay) of sodium equilenin sulfate formed; (2) rate (slope) of sodium equilenin sulfate formation; (3) disintegration time; and (4) dissolution time in percent released at 15 minutes.

**Tablet Preparation:** Each formulation was prepared as a 4 Kg batch blend with general L.O.D. of 1.1-1.2% prior to tableting. Each batch was tabletted using a rotary tablet machine with ¼" (0.250 inch) round, standard concave plain punches. Tablet target weight was 100 mg, hardness of 5-7 Kp, and friability of <1%. Tablets used in the study were uncoated. Thirteen (13) batches of tablets were produced, Lots 1681-15-1 to -13, of which 11 batches, Lots 1681-15-1 to -11, comprised the lots studied by analysis using the Statgraphics® program. Lot 1681-15-12 (a 0.625 mg EE/0.6 mg MT tablet) and Lot 1681-15-13 (a 0.15 mg EE/0.6 mg MT, using Pharmatose® (lactose) DCL-15) served as controls for comparison. A table correlating the manufactured lot numbers with the Statgraphics® Worksheet numbers used for the analyses is provided as Table 5.

**Table 5. Correlation of Tablet Batch Manufactured Lot Numbers with Statgraphics® Worksheet Run Numbers Used in Analyses.**

| **Manufactured Lot Numbers** | **Statgraphics® Worksheet Numbers** |
|---|---|
| Lot 1681-15-1 | Trial 4 |
| Lot 1681-15-2 | Trial 9 |
| Lot 1681-15-3 | Trial 7 |
| Lot 1681-15-4 | Trial 2 |
| Lot 1681-15-5 | Trial 3 |
| Lot 1681-15-6 | Trial 10 |
| Lot 1681-15-7 | Trial 8 |
| Lot 1681-15-8 | Trial 5 |
| Lot 1681-15-9 | Trial 1 |
| Lot 1681-15-10 | Trial 6 |
| Lot 1681-15-11 | Trial 11 |
| Lot 1681-15-12 | Control (0.625 mg EE/0.6 mg MT) |
| Lot 1681-15-13 | Control (0.15 mg EE/0.6 mg MT, DCL-15 Lactose) |

**Tablet Storage Conditions:** Tablet samples were stored in open-dish in controlled environmental chambers at accelerated conditions of 40° C/75% RH. Samples were withdrawn for evaluation at Initial (T₀), 2-Week and 4-Week time points.

**Tablet Characterization:** Assay results were provided by Analytical Development, Solvay Pharmaceuticals, Marietta, using an established HPLC method. Disintegration was performed by Pharmaceutical Development, Solvay Pharmaceuticals, Marietta, by USP/NF Method <701>, basket-rack assembly with discs, in a medium of 900 mL Purified Water at 37 ± 2°C. Results were reported as the average of six individual tablets. Dissolution determinations were performed by Analytical Development, Solvay Pharmaceuticals, Marietta, by USP/NF Method <711>, Apparatus 1 (baskets), 100 RPM, in 500 mL Purified Water at 37 ± 2°C. Aliquots were withdrawn and volume replaced at 15, 30, 45, 60, and 90 minutes. Results were reported as the Average (% Relative Standard Deviation) of six individual tablets. Results presented in this report focus on the 15-minute test sample.

### Results:

**Effect of Factors on Sodium Equilenin Sulfate Formation:** Reducing the quantity of sodium equilenin sulfate, a degradation product of sodium equilin sulfate formed in the individual formulation, is desirable. Data from analysis of the 4-Week samples indicates that the level of crospovidone has the greatest influence on the formation of sodium equilenin sulfate.

**Results at 2-Weeks at 40°C/75% RH Open-Dish Samples for Sodium Equilenin Sulfate Formation:** The analysis for the 2-Week open-dish samples stored at 40°C/75%RH for percent sodium equilenin sulfate content continued to show a strong influence by the level of crospovidone (XPVP). In these samples, an increase in the level of crospovidone from 0.0 to 6.0 mg/tablet showed a significant decrease in the quantity of equilenin formed, the reverse effect from that seen in the T₀ samples. Increases in starch or sodium ascorbate (NaAscorb) levels had less effect. An ANOVA of the 2-Week data indicated an improved fit of the model to the data, resulting in a higher correlation coefficient (R²adjusted = 45.85%) than was seen in the initial (T₀) data. The regression equation for the fitted model at 2-Weeks is: Sod_EQN_2Wk = 2.04205 + 0.000175(Starch Level) - 0.128417(XPVP Level) - 0.277(NaAscorb Level) - 0.00125833(Starch Level x XPVP Level) + 0.0147333(Starch Level x NaAscorb Level) - 0.0191111 (XPVP Level x NaAscorb Level). An interaction between the levels of starch and sodium ascorbate is noted, but the effect at this time point does not appear to be significant. With starch content held constant at a middle level, increases in crospovidone content generally result in decreases in equilenin formation. This effect is enhanced by also increasing sodium ascorbate levels, as seen in Figure 3. A cube plot of the data for the 2-Week samples, Figure 4, for the effects of all levels of the three factors suggests that the optimum combination or lowest formation of equilenin (0.68%) is achieved when starch is at minimum levels and sodium ascorbate and crospovidone are present at maximum levels in the formulation. Equilenin values for control Lots 1681-15-12 (0.625 mg EE) and -13 (DCL-15 lactose) at the 2-Week test point were 1.49% and 1.26%, respectively.

**Results at 4-Weeks at 40°C/75%RH Open-Dish Samples for Sodium Equilenin Sulfate Formation:** The analysis for the 4-Week open-dish samples stored at 40°C/75%RH for percent sodium equilenin sulfate content again exhibited a strong influence by the level of crospovidone (XPVP). In these samples, an increase in the level of crospovidone from 0.0 to 6.0 mg/tablet showed a significant decrease in the quantity of equilenin formed, the reverse effect from that seen in the T₀ samples and a similar effect to that observed in the 2-Week samples. Increases in sodium ascorbate levels also resulted in decreases in equilenin formation, but to a lesser extent than that from crospovidone. Increases in starch levels had less effect. An ANOVA of the 4-Week data indicated a reasonable fit of the model to the data, resulting in a correlation coefficient (R²adjusted = 44.96%) similar to the 2-Week data. The regression equation for the fitted model at 4-Weeks is: Sod_EQN_ 4Wk = 3.45359 + 0.0046625(Starch Level) - 0.282458(XPVP Level) - 0.925167(NaAscorb Level) + 0.00000416667(Starch Level x XPVP Level) + 0.0113167(Starch Level x NaAscorb Level) + 0.0373889(XPVP Level x NaAscorb Level). No potential interactions between factors studied were indicated by the analysis. A response plot for equilenin formation, holding sodium ascorbate content at a middle level, is presented in Figure 6. Note that the quantity of equilenin formed decreases as the content of crospovidone is increased, regardless of the starch content of the formulation. A cube plot of the data for 4-Week samples is provided in Figure 7. The plot suggests that formation of equilenin is minimized (0.707591%) when the starch content is minimized and the contents of both crospovidone and sodium ascorbate are maximized. This result is similar to that seen for the 2-Week sample data analysis. Equilenin values for control Lots 1681-15-12 (0.625 mg EE) and -13 (DCL-15 lactose) at the 4-Week test point were 2.04% and 1.57%, respectively.

**Results for Rate of Sodium Equilenin Sulfate Formation (Regression Slope) for 4-Week Samples Stored at 40°C/75%RH:** An estimate of the rate of sodium equilenin sulfate formation in each of the formulations was made by determining the slope of a linear regression equation obtained by graphing individual results of percent equilenin content versus time at T₀, 2-Week, and 4-Week time points. Values for Lots 1681-15-1 to -11 are presented in Attachment II; determinations for control Lots 1681-15-12 and -13 were not provided. Using the rate (slope) as a response variable in experimental design, an ANOVA of the data provided estimated effects of the factors studied for slope. Results showed that the fit to the model was calculated as R²adjusted = 45.26%. The regression equation for the fitted model of slope at 4-Weeks is: Slope = 0.848961 + 0.001175(Starch Level) - 0.0708333(XPVP Level) - 0.231133(NaAscorb Level) - 0.00000166667(Starch Level x XPVP Level) + 0.00281667(Starch Level x NaAscorb Level) + 0.00946667(XPVP Level x NaAscorb Level). The analysis showed that the effect of variation in the crospovidone level was statistically significant (p=0.0376). Increases in the crospovidone level resulted in a decrease in slope values, suggesting a decrease in the rate of equilenin formation for formulations containing higher amounts of crospovidone. Similarly, an increase in the sodium ascorbate level resulted in a decrease in slope value, but the degree of the effect was not statistically significant. Changes in starch level had a minimal effect on slope value. Representations of these relationships are presented in Figures 9 and 10. A review of the cube plot, Figure 10, shows that a minimum slope (0.16 %/Wk) for equilenin formation rate is achieved when both crospovidone and sodium ascorbate levels are high and starch level is at the minimum.

**Effect of Factors on Disintegration Time for EE (0.15 mg)/MT (0.6 mg) Combination Tablets:** An analysis of the estimated effects of the factors studied on disintegration times for tablets at the time of preparation (T₀) and after four weeks (4-Week) of storage in open-dish at 40°C/75%RH was performed. Experimental data for disintegration times in seconds at To and 4-Weeks are provided below:

**Initial Results (To) for Disintegration Time:** An analysis (ANOVA) for the estimated effects of factors at To showed only the level of crospovidone to have a statistically significant effect on disintegration time (p=0.0100). Increases in either the crospovidone or sodium ascorbate levels resulted in a decrease in the average disintegration time for the resultant tableted formulations. Changes in the starch level within the range studied had little effect on the To disintegration time. The adjusted R-squared (R²adjusted) statistic indicates that the model as fitted explains 66.05% of the variability in the disintegration time at To. The regression equation for the fitted model of disintegration time at 0-Weeks is: Disintrgr_Time_0wk = 432.75 - 0.8(Starch Level) - 33.6667(XPVP Level) - 54.6667(NaAscorb Level) + 0.341667(Starch Level x XPVP Level) + 1.23333(Starch Level x NaAscorb Level) - 3.66667(XPVP Level x NaAscorb Level). The values of the variables are specified in their original units. The relationship between levels of crospovidone and sodium ascorbate appears to be synergistic at To, as illustrated in Figure 12, a response surface plot holding starch content at a median level. An increase in crospovidone resulted in a decrease in disintegration time when sodium ascorbate is absent from the formulation. At the maximum level of crospovidone, the addition of sodium ascorbate further reduced the disintegration time observed. A review of the cube plot in Figure 13 indicated that the minimum disintegration time (116 seconds) for the uncoated tablet is achieved when the levels of crospovidone and sodium ascorbate are at maximum values and starch is absent from the formulation. Comparative To disintegration times for controls, Lots 1681-15-12 and -13, were reported as 101 seconds and 90 seconds, respectively.

**Results at 4-Weeks at 40°C/75%RH Storage Conditions for Disintegration Time:** An analysis (ANOVA) for the estimated effects of factors at 4-Week sample storage again showed only the level of crospovidone to have a statistically significant effect on disintegration time (p=0.0010). A combination of factors, starch level and crospovidone level, was seen to be nearly statistically significant (p=0.0527) at the 4-Week time point. An increase in the crospovidone level resulted in a decrease in the average disintegration time for the resultant tableted formulations. Changes in the starch level and sodium ascorbate level within the range studied had also resulted in decreased disintegration times, but to a lesser degree. The adjusted R-squared (R²adjusted) statistic indicates that the model as fitted explains 89.54% of the variability in the disintegration time at T_{4weeks}. The regression equation for the fitted model of disintegration time at 4-Weeks is: Disintrgr_Time_4wk = 1161.68 - 17.675(Starch Level) - 148.25(XPVP Level) - 144.333(NaAscorb Level) + 3.1 (Starch Level x XPVP Level) + 0.533333(Starch Level x NaAscorb Level) + 25.8889(XPVP Level x NaAscorb Level). The values of the variables are specified in their original units. From the results of the analysis, addition of sodium ascorbate to the formulation in the absence of starch caused a reduction in the disintegration time. When starch and sodium ascorbate were included, further reduction of the disintegration time was noted. However, the effects of starch could be negated or overshadowed by the addition of crospovidone, where disintegration time was reduced by the addition of starch in the absence of crospovidone, the effect of starch addition was not seen when crospovidone was included at its maximum level. This effect is presented in the response plot in Figure 15.

An interaction was noted between sodium ascorbate and crospovidone at the levels studied. Addition of crospovidone to the formulation in the absence of sodium ascorbate resulted in a more significant reduction in disintegration time than that seen when sodium ascorbate was included at its maximum level.

A review of the cube plot in Figure 16 indicated that the minimum disintegration time (272 seconds) for the uncoated tablet at the 4-Week time point was achieved when the level of crospovidone is at a maximum value, and sodium ascorbate and starch are absent from the formulation. It is noted that this time has increased over the minimum disintegration time determined for the T₀ samples. Comparative 4-Week disintegration times for Controls, Lot 1681-15-12 and -13, were reported as 735 seconds and 126 seconds, respectively.

Data from a comparison of the disintegration times for each lot of tablets, including Controls, at T₀ and at 4-Week time points are presented in Figure 17. Note that the X-axis is co-labeled with both the Statgraphics® Trial Number and the corresponding Lot Number used in this study. Figure 18 shows the percentage change for disintegration times in tablets of each formulation when results from T₀ (initial) are compared with those from 4-Week open-dish samples after exposure to 40°C/75%RH conditions. It can be seen that while all formulations showed some degree of increase in disintegration time, tablets from Lots 1681-15-1, -2, -4 and -12 (Control, 0.625 mg) exhibited the greatest percentage increase.

### Example 3 - Effect of Factors on Dissolution Release Rate for EE (0.15 mg)/MT (0.6 mg) Combination Tablets:

**Initial Results (To) for Dissolution of Esterified Estrogens (EE) at 15 minutes:** An analysis (ANOVA) for the estimated effects of factors at T₀ on the dissolution release characteristics of EE from each of the formulations revealed two effects with statistical significance, starch level (p=0.0412) and crospovidone level (p=0.0360). The adjusted R-squared (R²adjusted) statistic shows that the model explains 61.27% of the observed variability at this time point. The regression equation for the fitted model of dissolution release at T₀ is: EEDsoln_0wk_15min = 49.5068 + 1.8875(Starch Level) + 7.375(XPVP Level) + 8.56667(NaAscorb Level) - 0.208333(Starch Level x XPVP Level) + 0.0366667(Starch Level x NaAscorb Level) - 1.05556(XPVP Level x NaAscorb Level). The values of the variables are specified in their original units. A review of the Main Effects Plot for EE dissolution (0-Week, 15-minute test sample), Figure 19, shows that an increase in the level of either starch or crospovidone resulted in an increase in dissolution release of the EE component. Increases in sodium ascorbate also resulted in an increase in dissolution release, but to a lesser extent. A maximum rate of dissolution for the EE component (0-Week, 15-minute test sample), occurs at the combination of maximum levels of starch, crospovidone and sodium ascorbate (111.0%). In comparison, Controls, Lot 1681-15-12 and -13, provided values for dissolution at this test station of 99.6% and 105.2%, respectively.

**Results at 4-Weeks at 40°C/75%/RH Storage Conditions for Dissolution of Esterified Estrogens (EE) at 15 minutes:** An analysis (ANOVA) for the estimated effects of factors at 4-Weeks on the dissolution release characteristics of EE from each of the formulations again revealed two effects with statistical significance, starch level (p=0.0074) and crospovidone level (p=0.0020). The adjusted R-squared (R²adjusted) statistic shows that the model explains 89.51% of the observed variability at this time point. The regression equation for the fitted model of dissolution release at 4-Weeks is: EEDsoln_4wk_15min = 43.7136 + 0.46875(Starch Level) + 6.24583(XPVP Level) - 0.75(NaAscorb Level) + 0.0645833(Starch Level x XPVP Level) + 0.485(Starch Level x NaAscorb Level) - 2.66111 (XPVP Level x NaAscorb Level). The values of the variables are specified in their original units. A review of the Main Effects Plot for EE dissolution (4-Week, 15-minute test sample), Figure 20, shows that an increase in the level of either starch or crospovidone resulted in an increase in dissolution release of the EE component and is similar to the effects seen at T₀. Increases in sodium ascorbate resulted, however, in a nonsignificant decrease in dissolution release. Potential interactions are noted between starch and sodium ascorbate and between starch and crospovidone with respect to dissolution release of the EE component at 4-Weeks. As represented in Figure 21, the effects of starch level and crospovidone content appear to be additive with respect to dissolution release rate of the EE component at 4-Weeks. With sodium ascorbate held at the median level, an increase in the starch content results in an increase in dissolution rate, which is further increased at any point by the addition of crospovidone to the formulation. A review of the cube plot for this sample point, Figure 22, shows that the maximum dissolution rate (98.3 %) is achieved where starch and crospovidone levels are highest and sodium ascorbate is absent from the formulation.

**Initial Results (T₀) for Dissolution of Methyltestosterone (MT) at 15 minutes:** An analysis (ANOVA) for the estimated effects of factors at To on the dissolution release characteristics of MT from each of the formulations revealed two effects with statistical significance, starch level (p=0.0479) and crospovidone level (p=0.0282). This result is similar to that found for the EE component at T₀. The adjusted R-squared (R²adjusted) statistic shows that the model explains 62.69% of the observed variability at this time point. The regression equation for the fitted model of dissolution release at T₀ is: MTDsoln_0wk_15min = 24.9705 + 2.69875(Starch Level) + 10.7125(XPVP Level) + 5.05(NaAscorb Level) - 0.357917(Starch Level x XPVP Level) + 0.121667(Starch Level x NaAscorb Level) - 0.405556(XPVP Level x NaAscorb Level). The values of the variables are specified in their original units. A review of the Main Effects Plot for MT dissolution (0-Week, 15-minute test sample), Figures 23, shows that an increase in the level of either starch or crospovidone resulted in an increase in dissolution release of the MT component, as was seen in the EE component. Increases in sodium ascorbate also resulted in an increase in dissolution release, but again, to a lesser extent. A maximum rate of dissolution for the MT component (0-Week, 15-minute test sample), occurs at the combination of maximum levels of starch, crospovidone and sodium ascorbate (107.8%). In comparison, Controls, Lot 1681-15-12 and -13, provided values for dissolution at this test station of 95.0% and 92.0%, respectively.

**Results at 4-Weeks at 40°C/75%RH Storage Conditions for Dissolution of Methyltestosterone (MT) at 15 minutes:** An analysis (ANOVA) for the estimated effects of factors at 4-Weeks on the dissolution release characteristics of MT from each of the formulations again revealed two effects with statistical significance, starch level (p=0.0031) and crospovidone level (p=0.0026). The adjusted R-squared (R²adjusted) statistic shows that the model explains 90.44% of the observed variability at this time point. The regression equation for the fitted model of dissolution release at 4-Weeks is: MTDsoln_4wk_15min = 14.4545 + 1.36875(Starch Level) + 8.87917(XPVP Level) - 3.75(NaAscorb Level) - 0.03625(Starch Level x XPVP Level) + 0.631667(Starch Level x NaAscorb Level) - 3.27222(XPVP Level x NaAscorb Level). The values of the variables are specified in their original units. A review of the Main Effects Plot for MT dissolution (4-Week, 15-minute test sample), Figure 24, shows that an increase in the level of either starch or crospovidone resulted in an increase in dissolution release of the MT component and is similar to the effects seen at T₀. Similar to the EE component data, increases in sodium ascorbate resulted in a nonsignificant decrease in dissolution release of the MT component. Potential interactions are noted between starch and sodium ascorbate and between crospovidone and sodium ascorbate with respect to dissolution release of the MT component at 4-Weeks. As represented in Figure 25, the effects of starch level and crospovidone content appear to be additive with respect to dissolution release rate of the MT component at 4-Weeks. These results are similar to those for the 4-Week EE samples. With sodium ascorbate held at the median level, an increase in the starch content results in an increase in dissolution rate, which is further increased at any point by the addition of crospovidone to the formulation. A review of the cube plot for this sample point, Figure 26, shows that the maximum dissolution rate (90.7%) is achieved where starch and crospovidone levels are highest and sodium ascorbate is absent from the formulation.

### Summary of Results:

A summary of results of factor effects indicated to minimize or maximize, as appropriate, the measured response variables is presented in Table 5 below.

**Table 5. Comparison table of effects of Factors (independent variables) on measured Responses (dependent variables) in 4-Week open-dish samples stored at 40°C/75%RH. Samples are part of EtabDraft20050519 Design of Experiment for uncoated esterified estrogens (0.15 mg)/methyltestosterone (0.6 mg) tablet formulations, Reference Lots 1681-15-1 through -11.**

| **(Y) Response** | **Optimum Response** | **(Xᵢ) Main Factors** | **Optimum Levels** |
|---|---|---|---|
| Quantity of Sodium Equilenin Sulfate formed. | Minimize | Starch level Crospovidone level Sodium | Low (0 mg/tab) High (6 mg/tab) High (1.5 mg/tab) |
| Slope (rate) of Sodium Equilenin Sulfate formation. | Minimize | Starch level Crospovidone level Sodium | Low (0 mg/tab) High (6 mg/tab) High (1.5 mg/tab) |
| Dissolution % Release of Esterified Estrogens in 15 minutes. | Maximize | Starch level Crospovidone level Sodium | High (20 mg/tab) High (6 mg/tab) Low (0 mg/tab) |
| Dissolution % Release of Methyltestosterone in 15 minutes. | Maximize | Starch level Crospovidone level Sodium | High (20 mg/tab) High (6 mg/tab) Low (0 mg/tab) |
| Disintegration Time of Uncoated Esterified Estrogens/Methyltestosterone | Minimize | Starch level Crospovidone level Sodium | Low (0 mg/tab) High (6 mg/tab) Low (0 mg/tab) |

### Conclusions:

The purpose of this study was to investigate the effects of various levels of partially pregelatinized starch, crospovidone and sodium ascorbate on the characteristics of tablets containing a combination of esterified estrogens/methyltestosterone in a controlled stability study under accelerated conditions. These three factors were studied because of their widely accepted properties in direct tableting applications. Partially pregelatinized starch (Starch 1500®) functions as a binder and moisture scavenger; crospovidone is recognized as a superdisintegrant, and sodium ascorbate serves as an antioxidant in many formulations. Characteristics studied included quantity and rate of sodium equilenin sulfate formation, tablet disintegration time and tablet dissolution rate.

The study was performed using an experimental design to evaluate uncoated tablets of 11 formulations, plus two additional formulations that served as controls, which were stored under accelerated conditions of 40° C/75% RH in open-dish for periods up to four weeks.

While multiple characteristics were evaluated, the primary focus was on the stability of the sodium equilin sulfate component and its degradation product, sodium equilenin sulfate. A goal of the study was minimizing the quantity and rate of formation of sodium equilenin sulfate. Analyses suggested that the minimum rate of formation could be achieved with a formulation containing crospovidone 6.0 mg/tablet, sodium ascorbate 1.5 mg/tablet, and no partially gelatinized starch. In general, crospovidone content was the most significant factor in the study of effects.

The analyses also predicted that the use of this formulation would affect both disintegration time and dissolution release rates for both esterified estrogens and methyltestosterone components. A formulation containing crospovidone 6.0 mg/tablet, sodium ascorbate 1.5 mg/tablet, and no starch is estimated to cause a slight increase in disintegration time from the optimum for 4-Week samples stored at accelerated conditions, which is from approximately 272 seconds to 288 seconds, a 5.9% increase.

The predicted impact of this formulation on dissolution release rate for both active components is more significant. Dissolution rates at the 15-minute test point may decrease from an estimated optimum of approximately 98.3% to 56.1 % for this formulation, a 42.9% decrease.

All references, including publications, patent applications, and patents cited herein are hereby incorporated by reference to the same extent as if each reference were set forth in its entirety herein.

The use of the terms "a" and "an" and "the" and similar referents in the context of this disclosure (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., such as, preferred, preferably, particularly) provided herein, is intended merely to further illustrate the content of the disclosure and does not pose a limitation on the scope of the claims. No language in the specification should be construed as indicating any non-claimed element as essential to the practice of the claimed invention.

Alternative embodiments of the claimed invention are described herein, including the best mode known to the inventors for carrying out the claimed invention. Of these, variations of the disclosed embodiments will become apparent to those of ordinary skill in the art upon reading the foregoing disclosure. The inventors expect skilled artisans to employ such variations as appropriate, and the inventors intend for the claimed invention to be practiced otherwise than as specifically described herein.

Accordingly, the claimed invention includes all modifications and equivalents of the subject matter recited in the claims appended hereto as permitted by applicable law. Moreover, any combination of the above-described elements in all possible variations thereof is encompassed by the claimed invention unless otherwise indicated herein or otherwise clearly contradicted by context.

The use of individual numerical values are stated as approximations as though the values were preceded by the word "about" or "approximately." Similarly, the numerical values in the various ranges specified in this application, unless expressly indicated otherwise, are stated as approximations as though the minimum and maximum values within the stated ranges were both preceded by the word "about" or "approximately." In this manner, variations above and below the stated ranges can be used to achieve substantially the same results as values within the ranges. As used herein, the terms "about" and "approximately" when referring to a numerical value shall have their plain and ordinary meanings to a person of ordinary skill in the art to which the disclosed subject matter is most closely related or the art relevant to the range or element at issue. The amount of broadening from the strict numerical boundary depends upon many factors. For example, some of the factors which may be considered include the criticality of the element and/or the effect a given amount of variation will have on the performance of the claimed subject matter, as well as other considerations known to those of skill in the art. As used herein, the use of differing amounts of significant digits for different numerical values is not meant to limit how the use of the words "about" or "approximately" will serve to broaden a particular numerical value. Thus, as a general matter, "about" or "approximately" broaden the numerical value. Also, the disclosure of ranges is intended as a continuous range including every value between the minimum and maximum values plus the broadening of the range afforded by the use of the term "about" or "approximately." Thus, recitation of ranges of values herein are merely intended to serve as a shorthand method of referring individually to each separate value falling within the range, unless otherwise indicated herein, and each separate value is incorporated into the specification as if it there individually recited herein.

It is to be understood that any ranges, ratios and ranges of ratios that can be formed by, or derived from, any of the data disclosed herein represent further embodiments of the present disclosure and are included as part of the disclosure as though they were explicitly set forth. This includes ranges that can be formed that do or do not include a finite upper and/or lower boundary. Accordingly, a person of ordinary skill in the art most closely related to a particular range, ratio or range of ratios will appreciate that such values are unambiguously derivable from the data presented herein.

## Claims

1. A pharmaceutical composition comprising at least one esterified estrogen, methyltestosterone and a moisture scavenging agent.

2. The composition according to claim 1 wherein the at least one esterified estrogen is selected from the group consisting of sodium equilin sulfate and sodium estrone sulfate.

3. The composition according to any of claims 1 or 2, wherein a therapeutically effective amount of the at least one esterified estrogen is present in the composition after 180 days after the composition is made.

4. The composition according to any of claims 1 to 3, wherein 85% of the at least one esterified estrogen is present in the composition after 180 days after the composition is made.

5. The composition according to any of claims 1 to 4, wherein the moisture scavenging agent is selected from the group consisting of crospovidone or starch.

6. The composition according to any of claims 1 to 5, wherein the composition is a solid oral pharmaceutical dosage form selected from the group consisting of tablets, immediate release tablets, suspension tablets, bite suspension tablets, rapid dispersion tablets, chewable tablets, effervescent tablets, bilayer tablets, caplets, capsules, hard gelatin capsules, powders, packaged powders, dispensable powders or effervescent powders, lozenges, sachets, cachets, troches, pellets, granules, microgranules, encapsulated microgranules or powder aerosol formulations.

7. The composition according to any of claims 1 to 6, wherein the composition has a disintegration time of 100 seconds to 500 seconds or 120 seconds to 455 seconds at 15 minutes for a sample that is 0 weeks old; 300 seconds to 1200 seconds, or 310 seconds to 1185 seconds, at 15 minutes for a sample that is 4 weeks old.

8. A pharmaceutical composition comprising at least one esterified estrogen selected from the group consisting of sodium equilin sulfate and sodium estrone sulfate, methyltestosterone and a moisture scavenging agent.

9. The composition according to claim 8, wherein a therapeutically effective amount of the at least one esterified estrogen is present in the composition after 180 days after the composition is made.

10. The composition according to any of claims 8 or 9, wherein 85% of the at least one esterified estrogen is present in the composition after 180 days after it is made.

11. The composition according to any of claims 8 to 10, wherein the moisture scavenging agent is selected from the group consisting of crospovidone or starch.

12. The composition according to any of claims 8 to 11, wherein the composition is a solid oral pharmaceutical dosage form selected from the group consisting of tablets, immediate release tablets, suspension tablets, bite suspension tablets, rapid dispersion tablets, chewable tablets, effervescent tablets, bilayer tablets, caplets, capsules, hard gelatin capsules, powders, packaged powders, dispensable powders or effervescent powders, lozenges, sachets, cachets, troches, pellets, granules, microgranules, encapsulated microgranules or powder aerosol formulations.

13. The composition according to any of claims 8 to 12, wherein the composition has a disintegration time of 100 seconds to 500 seconds or 120 seconds to 455 seconds at 15 minutes for a sample that is 0 weeks old; 300 seconds to 1200 seconds, or 310 seconds to 1185 seconds, at 15 minutes for a sample that is 4 weeks old.

14. A pharmaceutical composition comprising at least sodium equilin sulfate, sodium estrone sulfate, methyltestosterone and crospovidone.

15. The composition according to claim 14, further comprising sodium ascorbate or starch.

16. The composition according to any of claims 14 or 15, wherein the composition is a solid oral pharmaceutical dosage form selected from the group consisting of tablets, immediate release tablets, suspension tablets, bite suspension tablets, rapid dispersion tablets, chewable tablets, effervescent tablets, bilayer tablets, caplets, capsules, hard gelatin capsules, powders, packaged powders, dispensable powders or effervescent powders, lozenges, sachets, cachets, troches, pellets, granules, microgranules, encapsulated microgranules or powder aerosol formulations.

17. The composition according to any of claims 14 to 16, wherein the composition has a disintegration time of 100 seconds to 500 seconds or 120 seconds to 455 seconds at 15 minutes for a sample that is 0 weeks old; 300 seconds to 1200 seconds, or 310 seconds to 1185 seconds, at 15 minutes for a sample that is 4 weeks old.

18. Use of a composition according to any of claims 1 to 17 for the manufacture of a medicament for the treatment or prevention of menopause and the symptoms thereof, such as vasomotor symptoms, myocardial infarction, stroke, invasive breast cancer, pulmonary emboli, sexual dysfunction and deep vein thrombosis.
